# EUROPEAN PATENT APPLICATION

(11) **EP 3 196 295 A1**
(43) Date of publication of application: **26.07.2017**
(21) Application number: 16152655.3
(22) Date of filing: 25.01.2016
(51) Int. Cl.: C12N 5/071

(54) **METHOD OF PRODUCING RENAL CELLS FROM FIBROBLASTS**

(71) Applicant: Albert-Ludwigs-Universität Freiburg, 79085 Freiburg (DE)
(72) Inventor: LIENKAMP, Soeren, 79104 Freiburg (DE); KAMINSKI, Michael, 79104 Freiburg (DE); ARNOLD, Sebastian, 79111 Freiburg (DE); WALZ, Gerd, 79104 Freiburg (DE)
(74) Representative: Kalhammer, Georg

(57) **Abstract**

The present invention relates to a method for producing renal cells, comprising overexpressing *Emx2, Hnf1b, Hnf4a* and *Pax8* in fibroblasts.

## Description

### BACKGROUND

End-stage renal disease is a major public health burden with a growing incidence of 100,000 new cases per year in the US¹. Therefore, the *in vitro* generation of renal cells represents a major challenge for kidney disease related research and regenerative medicine.

A number of studies have directed pluripotent cells towards a renal cell fate *in vitro.* Embryonic stem cells or induced pluripotent cells (iPSCs) that were cultured in the presence of defined growth factor cocktails or chemical compounds acquired renal cell characteristics²⁻⁶. These multistep protocols aimed to recapitulate the path of renal embryonic differentiation^{3, 6} and generated self-organizing kidney-like organoids that contained segmented nephrons^{2,4}, which were surrounded by endothelial cells and renal interstitium⁵. In a different approach, bone-marrow-derived mesenchymal stem cells were conditioned by cell-free extracts from a proximal tubule cell line to form tubule-like cells *in vitro*⁷. However, all of these approaches depend on the availability of pluri- or multipotent stem cell populations, and include intricate differentiation protocols. In addition, the use of human ESCs raises ethical concerns, and the tumorigenic risk of iPSC-derived cells currently limits their clinical use⁸.

Direct reprogramming is an alternative approach to generate desired cell types *in vitro*⁹. Differentiated cells, such as fibroblasts, can be reprogrammed by defined transcription factors into organ-specific cell types bypassing a pluripotent stage. Fibroblasts have been directly reprogrammed to neurons¹⁰, oligodendrocytes^{11,12}, cardiomyocytes¹³, hepatocytes¹⁴⁻¹⁶ or Sertoli cells¹⁷, but not yet to any renal cell type. Previously, the expression of six transcription factors, *Six1, Six2, Osr1, Eya1, Hoxa11, Snai2,* in combination with valproic acid treatment induces de-differentiation of a proximal tubule cell line into a precursor state¹⁸. However, this approach requires viable renal tubule cells, thus limiting its use in the majority of clinical settings of kidney disease. Direct reprogramming of fibroblasts towards a mature renal cell type has not been reported to date.

### SUMMARY OF THE INVENTION

The inventors identified four transcription factors - *Emx2, Hnf1b, Hnf4a* and *Pax8 -* that convert embryonic and adult fibroblasts to induced renal tubular epithelial cells, which were termed iRECs. Reprogrammed iRECs resemble primary tubular cells in their mRNA-expression profile and show morphological and functional features of kidney tubule epithelia. They integrate into kidney organoids and form tubular structures in a decellularized kidney scaffold.

The present invention therefore relates to the following embodiments (1) to (16):
(1) A method for producing renal cells, comprising overexpressing *Emx2, Hnf1b, Hnf4a* and *Pax8* in differentiated cells.
(2) The method of item (1), wherein the renal cells are renal tubular cells.
(3) The method of item (1), wherein the renal cells are renal tubular epithelial cells.
(4) The method of any one of the preceding items, wherein the differentiated cells are mammalian cells.
(5) The method of any one of the preceding items, wherein the differentiated cells are human cells.
(6) The method of any one of the preceding items, wherein the differentiated cells are fibroblasts.
(7) The method of item (6), wherein the fibroblasts are embryonic fibroblasts.
(8) The method of item (6), wherein the fibroblasts are adult fibroblasts.
(9) The method of any one of the preceding items, wherein said overexpressing comprises
   (a) providing one or more nucleic acids encoding Emx2, Hnf1b, Hnf4a and Pax8; (b) introducing said one or more nucleic acids into the differentiated cells so as to obtain transduced or transfected differentiated cells; and (c) culturing said transduced or transfected differentiated cells under conditions that allow overexpression of *Emx2, Hnf1b, Hnf4a* and *Pax8.*
(10) The method of item (9), wherein said one or more nucleic acids are plasmids or vectors comprising a nucleotide sequence encoding *Emx2, Hnf1b, Hnf4a, Pax8* or a combination thereof, operably linked to a promoter capable of inducing overexpression of the encoded genes.
(11) The method of any one of the preceding items, wherein said *Emx2* comprises the nucleotide sequence as shown in SEQ ID NO:1 or a functional fragment thereof.
(12) The method of any one of the preceding items, wherein said *Hnf1b* comprises the nucleotide sequence as shown in SEQ ID NO:2 or a functional fragment thereof.
(13) The method of any one of the preceding items, wherein said *Hnf4a* comprises the nucleotide sequence as shown in SEQ ID NO:3 or a functional fragment thereof.
(14) The method of any one of the preceding items, wherein said *Pax8* comprises the nucleotide sequence as shown in SEQ ID NO:4 or a functional fragment thereof.
(15) A renal cell obtainable by the method of any one of the preceding items.
(16) The use of the renal cell of item (15) for nephrotoxicity testing, pharmacological screening or disease modeling.

### BRIEF DESCRIPTION OF THE DRAWINGS

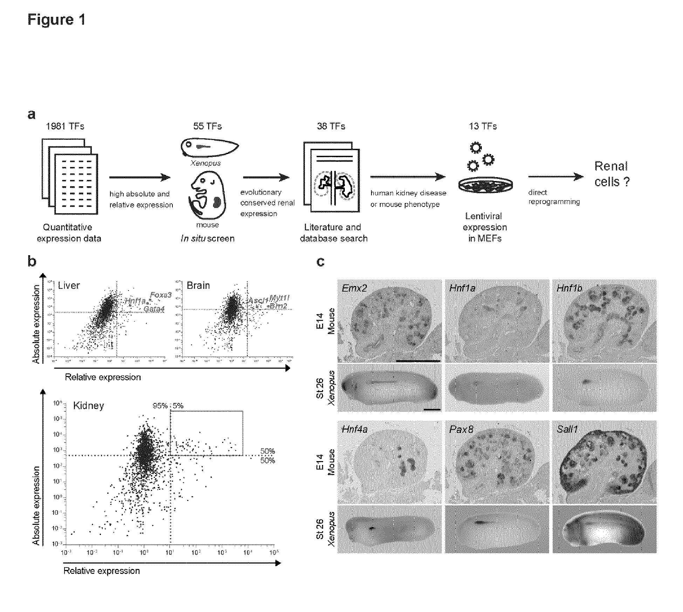

**Figure 1: Systematic selection criteria identify candidate reprogramming factors.**
   (**a**) Schematic illustrating the strategy and criteria used to identify renal reprogramming factors. TFs: transcription factors (**b**) Scatter-plot depicting the absolute and relative expression levels of transcription factors in liver, brain and kidney tissue²². Factors used for successful reprogramming to hepatocytes and neurons are highlighted in red. Dashed lines indicate the level of the 50th percentile of the absolute expression and the 95th percentile of relative expression. The area boxed in red includes the 55 candidate reprogramming factors for renal cell types. (**c**) Representative images of in situ hybridizations of the indicated genes on E14 mouse kidney sections and stage 26 *Xenopus* whole-mount embryos. Scale bars, 500 µm (**c**).

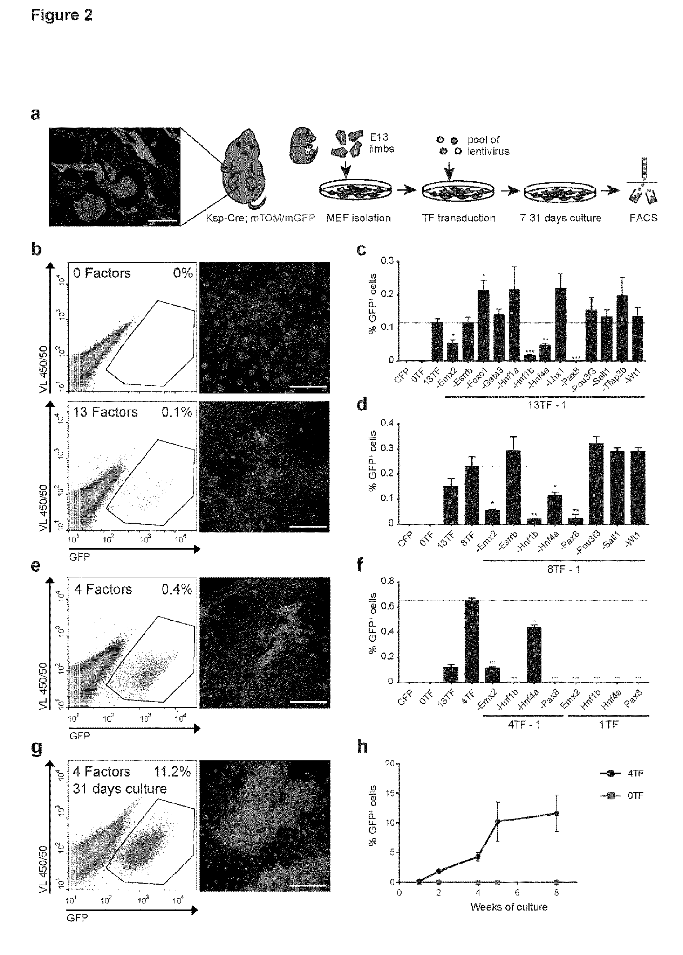

**Figure 2: Four transcription factors can induce renal cell fate in MEFs.**
   (**a**) Schematic of the experimental procedure used to identify renal reprogramming factors. A kidney section from adult KSP-Cre; mTOM/mGFP reporter mice shows membrane-GFP expression (mGFP) in the renal tubules and membrane-tomato red fluorescence (mTOM) in the remaining tissue. (**b**) MEFs derived from limbs of reporter mice were left untreated (0 factors) or transduced with the pool of 13 transcription factors (13TF) and analyzed by flow cytometry (FC) for the presence of GFP positive (GFP⁺) cells 7 days after viral transduction. Confocal images show GFP⁺ cells (green), mTOM⁺ cells (red), and nuclear staining with Hoechst (blue) (**c**) Percentages of GFP⁺ cells in different 13TF-1 combinations were assessed by FC; 0TF: untreated MEFs; CFP: cyan fluorescent protein. The broken line marks the average percentage of GFP⁺ cells in the pool of 13TFs. (**d**) Percentage of GFP⁺ cells in different 8TF-1 combinations as assessed by FC. (**e**) FC analysis and representative confocal image of GFP⁺ cells transduced with the pool of 4 TFs *(Emx2, Hnf1b, HNf4a, Pax8).* (**f**) FC analysis of GFP⁺ cells in MEFs overexpressing the 4TFs, combinations of three TFs, or single factors. (**g**) FC analysis and representative confocal image of 4TF treated cells after 31 days of culture. (**h**) Time course of GFP⁺ cells as determined by FC after culture for the indicated period of time. Error bars: standard error of the mean (SEM), Asterisks indicate significant differences to the positive control (13TF, 8TF and 4TF) as assessed by Student's unpaired t-test, n=3, *** p<0.001, ** p<0.01, * p<0.05, Scale bars, 100 µm (**a**,**b**,**e**,**g**)

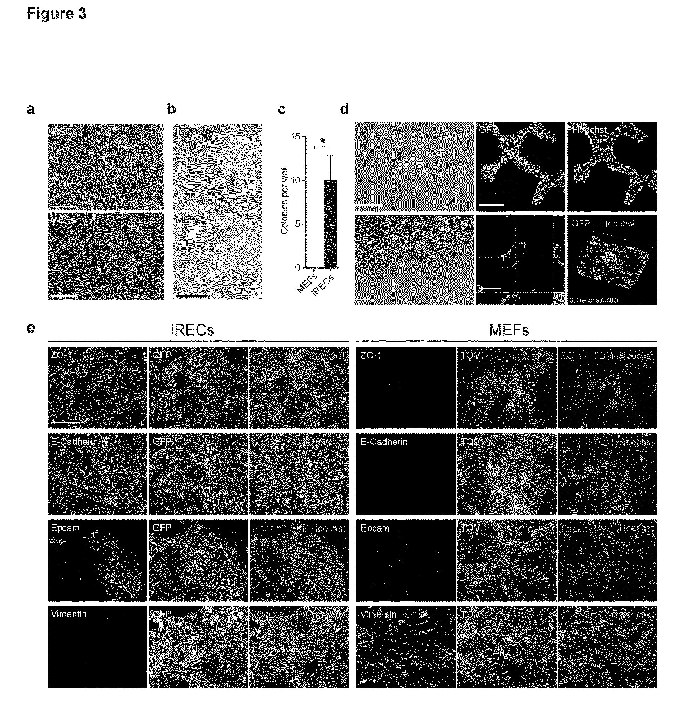

**Figure 3: iRECs exhibit epithelial properties.**
   (**a**) Phase contrast images of iRECs and MEFs show epithelial and mesenchymal cell morphologies, respectively. (**b**) Colony formation of iRECs and MEFs was assessed by crystal violet staining. (**c**) Quantification of colonies formed by MEFs and iRECs after 2 weeks of culture. (* p<0.05, student's t-test, n=3, Error bars: SEM) (**d**) Differential interference contrast (DIC) and confocal imaging: Sparsely seeded iRECs on Matrigel-coated dishes show branching tubular structures (upper panel); densely seeded iRECs display dome formation as observed in orthogonal view and by 3D reconstruction of confocal z-stacks (lower panel). (**e**) Immunofluorescence staining of the epithelial (ZO-1, E-Cadherin, Epcam) and mesenchymal (Vimentin) marker proteins in iRECs and MEFs. Scale bars, 50 µm (**a**,**e**), 1 cm (**b**), 100 µm (*d*).

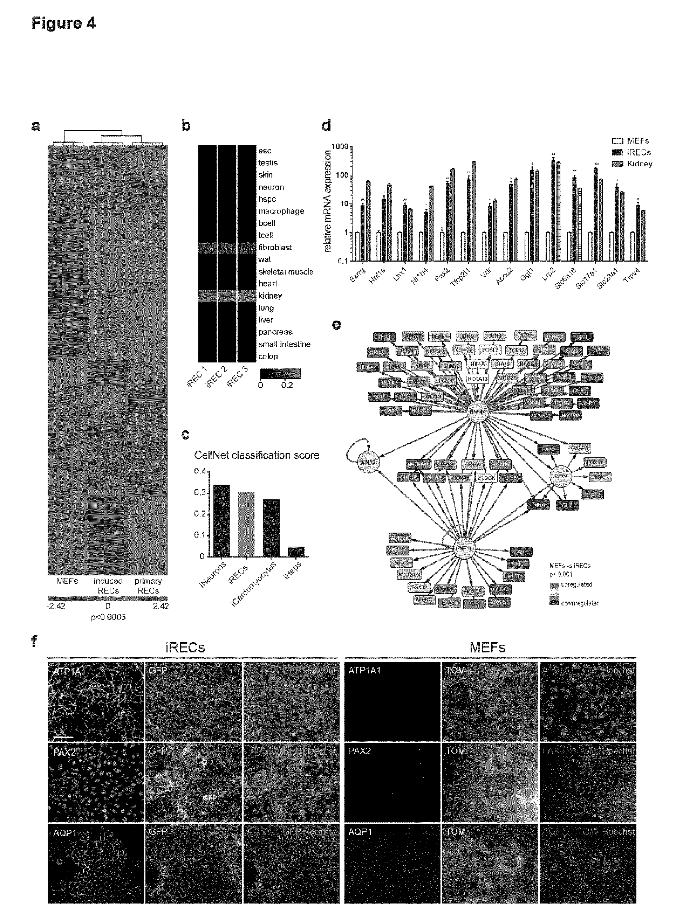

**Figure 4: iRECs resemble primary renal epithelial cells in their global gene expression pattern.**
   (**a**) Hierarchical clustering and heatmap image of cDNA microarray data comparing gene expression profiles of MEFs, iRECs and primary RECs isolated from Ksp-Cre; mTOM/mGFP kidneys (n=4 for each group). Blue indicates downregulation, red indicates upregulation. Clustering was performed for 1.5-fold up/downregulated genes of iRECs vs. MEFs (p<0.0005). (**b**) CellNet-generated heatmap with each row representing the indicated cell type or tissue and each column representing one of three independently reprogrammed iREC cultures. Values representing the classification score are color-coded (black to green). (**c**) Classification scores of previously published directly reprogrammed cell types and iRECs as determined by CellNet³⁰. (**d**) Relative mRNA expression levels of renal marker genes in MEFs, iRECs, and whole kidney lysates as determined by qRT-PCR. Significant differences were assessed by Student's unpaired t-test, n=3, *** p<0.001, ** p<0.01, * p<0.05; Error bars: SEM. (**e**) Transcriptional circuitry network of predicted target genes of iREC reprogramming factors (yellow circles). Blue indicates down-regulation, red indicates upregulation. (**f**) Immunofluorescence staining of the indicated renal tubular proteins in iRECs and MEFs. Scale bar, 50 µm (**f**).

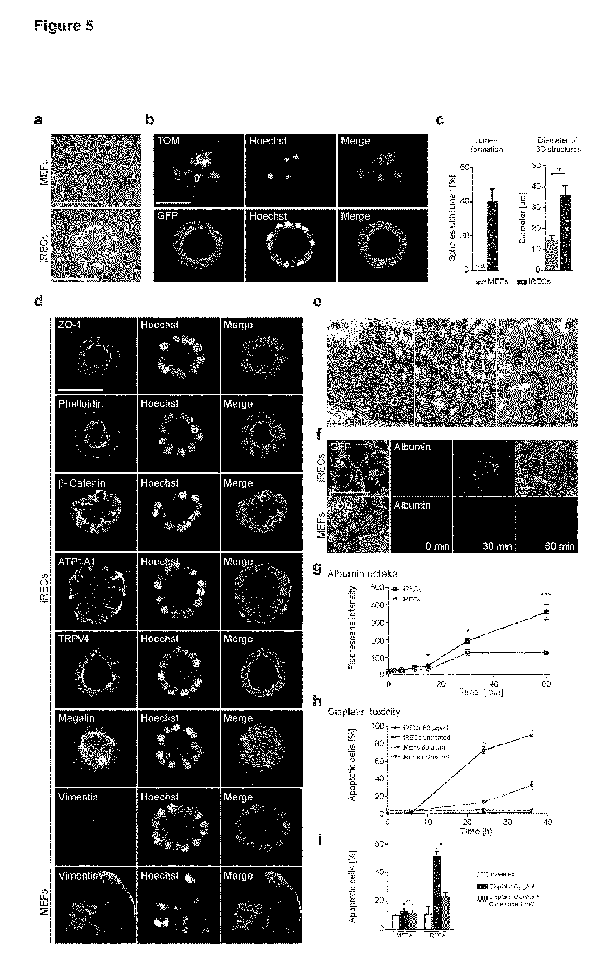

**Figure 5: iRECs show functional properties of renal tubular epithelial cells.**
   (**a**) Differential interference contrast (DIC) images of MEFs and iRECs grown in 3D Matrigel. (**b**) Confocal live-cell imaging of tomato red (TOM) expressing MEFs and GFP expressing iRECs grown in 3D Matrigel. Nuclei stained with Hoechst. (**c**) Quantification of lumen formation (%) and maximum diameter (µm) of 3D structures formed by MEFs (grey bars) and iRECs (black bars) in Matrigel. n.d.= not detectable. (**d**) Immunostaining of the indicated proteins in iRECs and MEFs grown in 3D Matrigel. Nuclei were stained with Hoechst. Representative confocal images show planes of maximum diameter of the spheres. (**e**) Transmission electron microscopy images of iRECs grown in 3D Matrigel. TJ: tight junction; M: microvilli; BML: basement membrane like matrix; N: nucleus. (**f**) Representative images of MEFs and iRECs incubated with alexa-647 labeled albumin for the indicated time-periods. (**g**) Quantification of albumin uptake in MEFs and iRECs over a 60 min time course. (**h**) Quantification of apoptotic cells (%) in cisplatin treated MEFs (red line) and iRECs (black line) or untreated controls. (**i**) Percentage of apoptotic cells in untreated, cisplatin only treated or cisplatin and cimetidine treated MEFs and iRECs. Error bars: SEM, Student's unpaired t-test, n=3, *** p<0.001, ** p<0.01, * p<0.05; Scale bars, 50 µm (**a**,**b**,**d**,**f**), 500 nm (**e**).

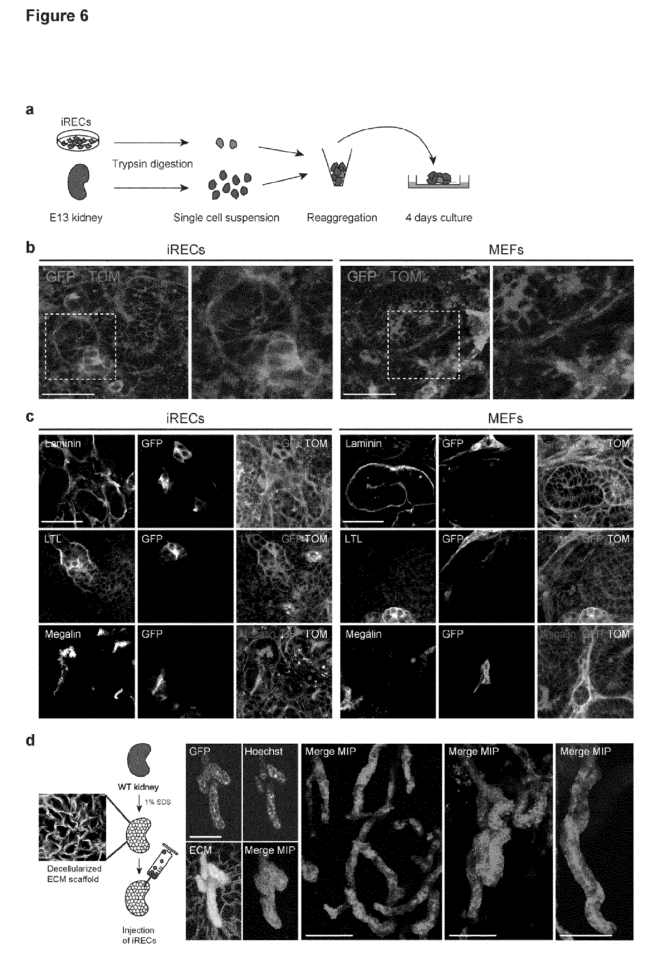

**Figure 6: iRECs integrate into reaggregated renal organoids and repopulate decellularized kidneys.**
   (**a**) Schematic illustration of the reaggregation assay. GFP⁺ iRECs and E13 TOM⁺ kidneys were trypsin digested, mixed in a 1:10 ratio and grown on a liquid-air interface culture. (**b**) Confocal live-imaging of renal organoids reaggregated with either KSP-Cre GFP⁺ iRECs or GFP transduced control MEFs. The white boxed area is shown enlarged in the right panel. (**c**) Immunostaining of kidney organoids reaggreagated with MEFs or iRECs for the indicated proteins. (**d**) Schematic illustration of the repopulation assay. Wild type kidneys were decellularized with 1% SDS and injected with iRECs; confocal images and 3D reconstructions of decullarized kidneys repopulated with KSP-Cre GFP⁺ iRECs. Nuclei were stained with Hoechst. ECM: extra cellular matrix, MIP: Maximum intensity projection of confocal z-stacks. Scale bars, 50 µm (**b**,**c**), 100 µm (**d**).

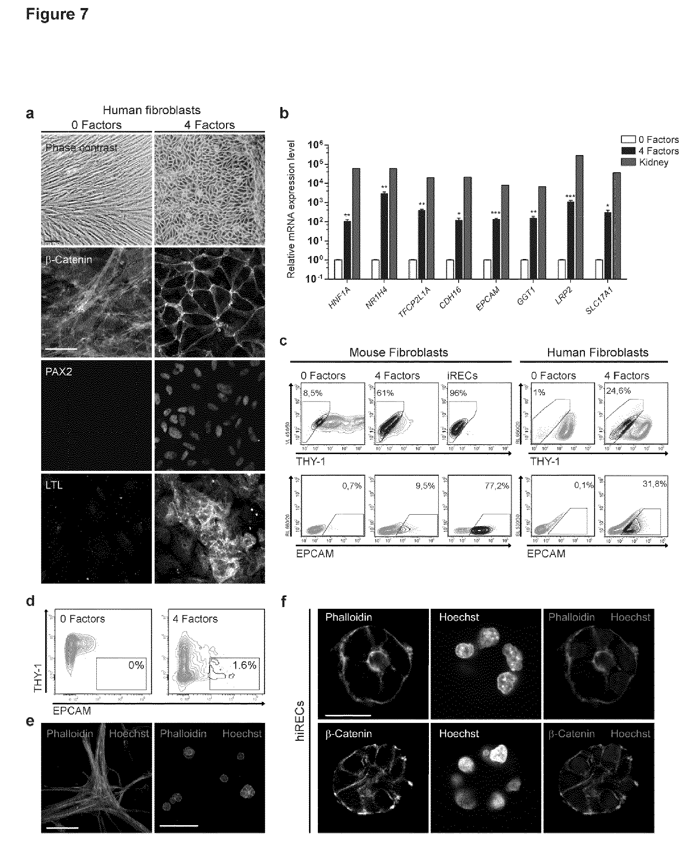

**Figure 7: Reprogramming of human fibroblasts.**
   (**a**) Phase contrast image and immunostaining for the indicated proteins in untreated and 4TF treated human fibroblasts. (**b**) Relative mRNA expression level of the indicated genes as determined by qRT-PCR. Significant differences were assessed by Student's unpaired t-test, n=3, *** p<0.001, ** p<0.01, * p<0.05; Error bars: SEM. (**c**) Percentage of THY-1- or EPCAM⁺ cells in mouse and human fibroblasts after treatment with 4TF as determined by flow cytometry. (**d**) Sorting strategy for THY-1⁻ and EPCAM⁺ cells in untreated and 4TF treated human fibroblasts. (**e**) Untreated human fibroblasts and human induced renal epithelial cells (h-iRECs) cultured in Matrigel. Shown are maximum intensity projections of confocal z-Stacks. (**f**) Matrigel grown h-iREC spheres stained for the indicated proteins. Scale bars, 50 µm (**a**), 100 µm (**e**), 20 µm (**f**).

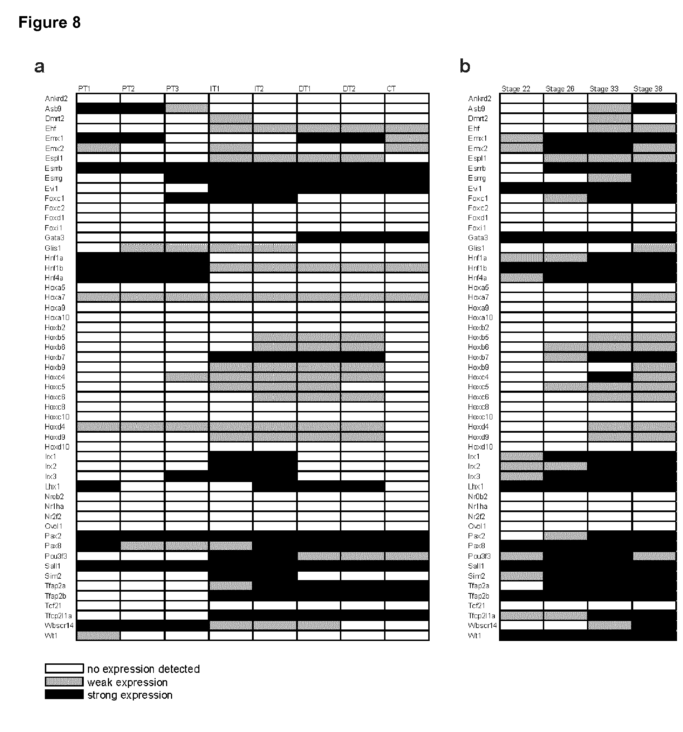

**Figure 8. Spatial and temporal expression analysis of candidate reprogramming factors in *Xenopus* and mouse embryos.**
   (**a**) Summary of whole mount in situ hybridization experiments: Spatial expression pattern. White, grey and black colored boxes indicate undetectable, weak and strong gene expression within the respective segment of the *Xenopus* pronephros. PT1, PT2, PT3: Proximal Tubule Segment 1, 2 and 3. IT1, IT2: Intermediate Tubule Segment 1 and 2. DT1, DT2: Distal Tubule Segment 1 and 2. CT: Connecting Tubule. (**b**) Summary of whole mount in situ hybridization experiments: Temporal expression pattern at stages 22, 26, 33 and 38 of *Xenopus* development. (**c**) Spatial expression pattern in mouse embryos as detected by WISH. (**d**) Temporal expression pattern in mouse embryos as detected by WISH.

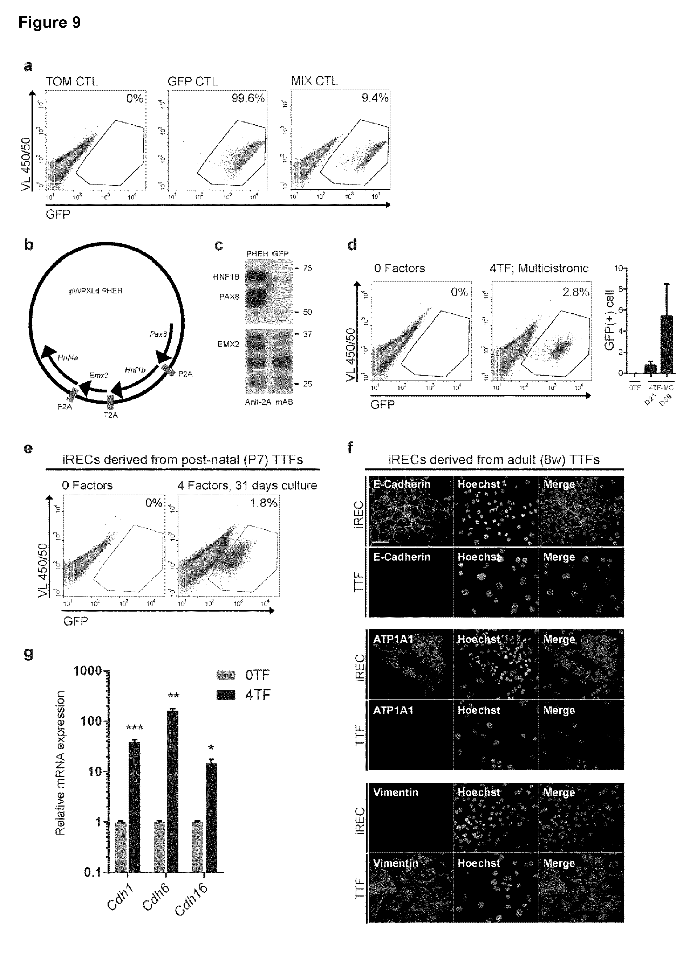

**Figure 9. iRECs can be induced with a multicistronic lentiviral vector and generated from adult tail tip fibroblasts.**
   (**a**) Control MEFs for FACS gate setting, TOM CTL: MEFs obtained from membrane-tomato red mice; GFP CTL: MEFs obtained from membrane-GFP mice; MIX CTL: mixture of membrane-tomato red MEFs and membrane-GFP MEFs. (**b**) Schematic vector map of the multicistronic construct showing the four reprogramming factors (Pax8, Hnf1 b, Emx2, Hnf4a) separated by the indicated 2A peptide sequences. pWPXLd was used as lentiviral backbone. (**c**) Expression control of the 2A-tagged transcription factors with an antibody against the 2A consensus motif. GFP transfected cells served as a control. (**d**) FACS analysis of GFP⁺ cells in KSP-Cre reporter MEFs treated with lentivirus harboring the multicistronic vector after 39 days of culture. (**e**) FACS analysis of GFP⁺ cells in KSP-Cre reporter post-natal tail tip fibroblasts (TTFs). (**f**) Immunostaining of iRECs derived from adult TTFs against the indicated proteins. (**g**) Relative mRNA expression levels of the indicated genes as determined by qRT-PCR in untreated and 4TF (Emx2, Hnf1b, Hnf4a, Pax8) treated MEFs. All data are represented as mean of 3 independent experiments. Error bars: SEM, Scale bars represent 50µm; Asterisks indicate significant differences as assessed by Student's unpaired t-test, *** p<0.001, ** p<0.01, * p<0.05, ns: non-significant, p>0.05.

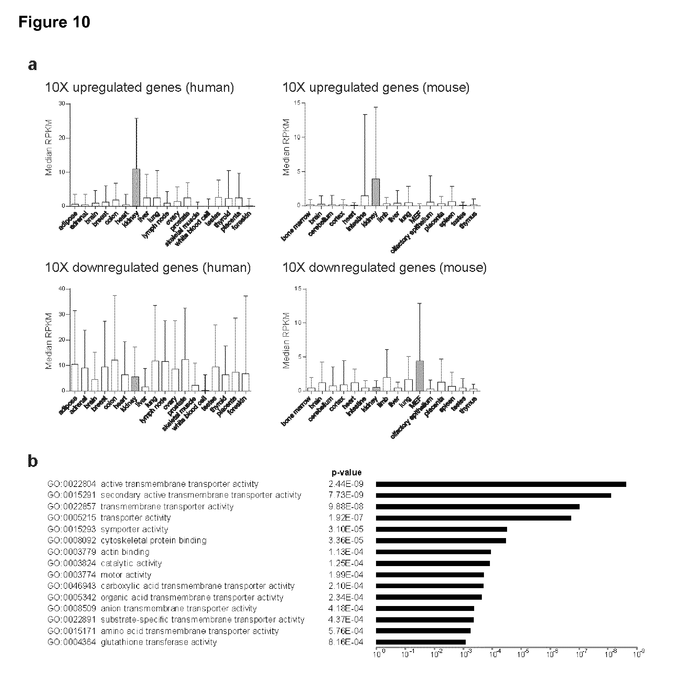

**Figure 10. Transcriptomic analysis of iRECs**
   (a) Gene ontology term analysis of 10-fold upregulated genes in iRECs compared to MEFs. (b) Median RPKM (Reads per kilobase per million mapped reads) of 10X upregulated genes in iRECs compared to MEFs in human and mouse RNAseq datasets of the indicated tissues. RNAseq data from (Cabili et al., 2011; Shen et al., 2012).

### DETAILED DESCRIPTION

The present invention relates to a method for producing renal cells, comprising overexpressing *Emx2, Hnf1b, Hnf4a* and *Pax8* in differentiated cells.

The term "differentiated cell" as used herein refers to a cell in the process of differentiating into a somatic cell lineage or having terminally differentiated. For example, embryonic cells can differentiate into an epithelial cell lining the intestine. Differentiated cells can be isolated from a fetus or a live born animal, for example.

Preferably, the differentiated cells are fibroblasts. Other suitable types of differentiated cells include cells of the mononuclear phagocyte system (MPS) (= Reticuloenothelial system) and other renal cell types.

### Fibroblasts

The term "fibroblast", as used herein, refers to a cell of mesenchymal origin. Fibroblasts are found in connective tissue. Fibroblasts synthesize actin-myosin filaments, the matrix elements (collagen, reticular and elastic fibers), and glycosaminoglycans and glycoproteins, which are secreted as amorphous intercellular substance. Fibroblasts include connective-tissue stem cells, matrix- and other protein-synthesizing cells, contractile cells, and phagocytic cells. Active fibroblasts are characterized by their abundant endoplasmic reticulum (ER), Golgi complex and ribosomes.

Fibroblasts play a particularly critical role during embryogenesis. Besides synthesizing proteins, they determine the structure of the skeleton, the location of muscle cells, the growth patterns of nerve fibers and the organization of the skin. Fibroblasts accomplish these organizational functions by attaching collagenous fibrils to embryonic cells and pulling them into the proper alignment to form parts of the developing organism. During human development and throughout adulthood, fibroblasts continue to synthesize and maintain both loose and dense types of connective tissue. They migrate in response to a number of chemoattractants such as lymphokines, cytokines and growth factors and constantly remodel and repair tissues by producing various degradative and synthetic enzymes, including collagenase, and products that may modulate the function of other cells including prostaglandins, tissue plasminogen activator (tPA), complement components and superoxide dismutase. The importance of fibroblasts may be attributed to their production of collagen, the predominant extracellular component of connective tissue and the most abundant protein in the human body.

The biology of fibroblasts and matrix proteins is discussed by Postlethwaite and Kang, In: Inflammation: Basic Principles and Clinical Correlates, 2d ed, Gallin et al. (1992) Raven Press, New York, pp 747-773.

In one embodiment, the fibroblasts are embryonic fibroblasts. The term "embryonic" as used herein refers to the age when the fibroblasts are obtained, i.e. the fibroblasts are obtained from an embryo, preferably at the age of E11.5-E14.5.
In another embodiment, the fibroblasts are adult fibroblasts. The term "adult" as used herein refers to the age when the fibroblasts are obtained, i.e. the fibroblasts are obtained from an adult subject. An adult subject in accordance with this invention has an age of more than 8 weeks.
In yet another embodiment, the fibroblasts are postnatal fibroblasts. Postnatal fibroblast are typically obtained from newborn subjects shortly after birth, e.g. within one week after birth, or at about one week after birth.

The fibroblast may be identified by immunocytochemistry using one or more of the following antibodies: (i) an antibody capable of binding to vimentin, (ii) an antibody capable of binding to prolyl-4-hydroxylase, and (iii) the antibody TE-7 (commercially available from EMD Millipore, Catalog No. CBL271; see also Haynes, B.F., et al. (1984). J. Exp. Med. 159(4):1149-1168).

In embodiment A, the fibroblast carries a surface antigen that is recognized by the antibody TE-7. In embodiment B, the fibroblast expresses vimentin. In embodiment C, the fibroblast expresses prolyl-4-hydroxylase. Further aspects of the invention are combinations of embodiments A and B, A and C, B and C, and A, B and C.

### Renal cells

The term "renal cell", as used herein, refers to a cell having the characteristics of a kidney cell or residing within the kidney or originating from the metanephric mesenchyme or the ureteric bud.
The renal cells obtained in accordance with this invention preferably express the genes *Pax2, Lhx1, Slc6a18, Slcl7a1* and/or *Lrp2,* and/or they show upregulation of one or more of these genes relative to the fibroblasts from which they originated.

Preferably, the renal cell is a renal tubular cell. More preferably, the renal cell expresses the marker protein "kidney-specific Cadherin", also termed Cadherin-16 (see also Thomson et al. (1995) J Biol Chem 270 (29): 17594-17601; and Shen et al. (2005) Mod Pathol. 18(7): 933-940 and references cited therein). Antibodies against Cadherin-16 are commercially available.

Most preferably, the renal cell is an epithelial renal cell, e.g. a renal tubular epithelial cell. The epithelial renal cells are typically characterized by the presence of the poteins ZO-1, E-cadherin (CDH1) and EpCAM at the membrane of the epithelial renal cells.

The epithelial renal cells preferably express *Cdh1,* which encodes E-cadherin, and *Cdh6,* and/or the expression of these genes is greater than that in the fibroblasts from which they originated.

In addition, the renal cells are preferably negative for vimentin, or at least the expression of vimentin in the renal cells is significantly lower than that in the fibroblasts from which they originated.

Unless indicated otherwise, all references to expression levels herein refer to a determination by quantitative Real-time PCR, as defined in the examples.

### Transcription factors

The **EMX2** gene encodes a homeobox-containing transcription factor that is the homolog to the 'empty spiracles' gene in Drosophila. Research on this gene in humans has focused on its expression in three tissues: dorsal telencephalon, olfactory neuroepithelium, and urogenetial system. It is expressed in the dorsal telencephalon during development in a low rostral-lateral to high caudal-medial gradient and is proposed to pattern the neocortex into defined functional areas. It is also expressed in embryonic and adult olfactory neuroepithelia where it complexes with eukaryotic translation initiation factor 4E (eIF4E) and possibly regulates mRNA transport or translation. In the developing urogenital system, it is expressed in epithelial tissues and is negatively regulated by HOXA10. Alternative splicing results in multiple transcript variants encoding distinct proteins.

The human ortholog of EMX2 protein has the amino acid sequence deposited under UniProtKB/Swiss-Prot accession number Q04743. Isoform 1 has the accession number Q04743-1, whereas isoform 2 has the accession number Q04743-2.
Isoform 1 is encoded by a cDNA sequence with the following details:

| | |
|---|---|
| LOCUS | NM_004098 2908 bp |
| DEFINITION | Homo sapiens empty spiracles homeobox 2 (EMX2), transcript variant 1, mRNA. |
| ACCESSION | NM_004098 |
| VERSION | NM_004098.3 GI:164607120 |

Isoform 2 is encoded by a cDNA sequence with the following details:

| | |
|---|---|
| LOCUS | NM_001165924 2723 bp |
| DEFINITION | Homo sapiens empty spiracles homeobox 2 (EMX2), transcript variant 2, mRNA. |
| ACCESSION | NM_001165924 |
| VERSION | NM_001165924.1 GI:260064071 |

Any nucleotide sequence encoding human EMX2, an ortholog thereof, or a functional fragment thereof, can be used in accordance with this invention. The nucleic acid encoding EMX2 may have a sequence identity of at least 80%, preferably of at least 90%, to the nucleotide sequence as shown in SEQ ID NO:1 [accession number NM_004098]; For reprogramming mouse fibroblasts the nucleotide sequence as shown under accession number NM_010132.2 can be used.

The **Hnf1b** gene encodes a member of the homeodomain-containing superfamily of transcription factors. The protein binds to DNA as either a homodimer, or a heterodimer with the related protein hepatocyte nuclear factor 1-alpha. The gene has been shown to function in nephron development, and regulates development of the embryonic pancreas. Mutations in this gene result in renal cysts and diabetes syndrome and noninsulin-dependent diabetes mellitus, and expression of this gene is altered in some types of cancer. Multiple transcript variants encoding different isoforms have been found for this gene.

The human ortholog of HNF1B has the amino acid sequence as defined under UniProtKB/Swiss-Prot accession number P35680. This includes the isoforms defined under accession numbers P35680-1, P35680-2, P35680-3, and P35680-4.

Corresponding mRNA sequences include, but are not limited to, the nucleotide sequences defined under accession numbers:
BC017714.1, XM_011546821.1, NM_006481.1, NM_000458.3, XM_011546819.1,
XM_011546822.1, XM_011525162.1, NM_001165923.3, XM_011525163.1,
XM_011525160.1, XM_011546823.1, XM_011546820.1, NM_001304286.1,
XM_011525161.1, and XM_011525164.1

Any nucleotide sequence encoding human HNF1B, an ortholog thereof, or a functional fragment thereof, can be used in accordance with this invention. The nucleic acid encoding HNF1B may have a sequence identity of at least 80%, preferably of at least 90%, to the nucleotide sequence as shown in SEQ ID NO:2 [accession number BC017714.1]. For reprogramming mouse fibroblasts the nucleotide sequence as shown under accession number NM_009330.2 can be used.

The **HNF4A** protein is a nuclear transcription factor which binds DNA as a homodimer. The encoded protein controls the expression of several genes, including hepatocyte nuclear factor 1 alpha, a transcription factor which regulates the expression of several hepatic genes. Mutations in this gene have been associated with monogenic autosomal dominant noninsulin-dependent diabetes mellitus type I. Alternative splicing of this gene results in multiple transcript variants encoding several different isoforms.

The human ortholog of HNF4A has the amino acid sequence as defined under UniProtKB/Swiss-Prot accession number P41235. This includes the isoforms defined under accession numbers P41235-1, P41235-2, P41235-3, P41235-4, P41235-5, P41235-6 and P41235-7.

Corresponding mRNA sequences include, but are not limited to, the nucleotide sequences defined under accession numbers:
NM_001287182.1, NM_001030003.2, NM_175914.4, XM_005260407.2, NM_001030004.2,
NM_001287183.1, NM_178849.2, XM_011528797.1, NM_000457.4, NM_001287184.1,
NM_178850.2, NM_001258355.1 and XM_011528798.1.

Any nucleotide sequence encoding human HNF4A, an ortholog thereof, or a functional fragment thereof, can be used in accordance with this invention. The nucleic acid encoding HNF1B may have a sequence identity of at least 80%, preferably of at least 90%, to the nucleotide sequence as shown in SEQ ID NO:3 [accession number NM_000457.4]. For reprogramming mouse fibroblasts the nucleotide sequence as shown under accession number NM_008261.2 can be used.

The **Pax8** gene encodes a member of the paired box (PAX) family of transcription factors. Members of this gene family typically encode proteins that contain a paired box domain, an octapeptide, and a paired-type homeodomain. This nuclear protein is involved in thyroid follicular cell development and expression of thyroid-specific genes. Mutations in this gene have been associated with thyroid dysgenesis, thyroid follicular carcinomas and atypical follicular thyroid adenomas. Alternatively spliced transcript variants encoding different isoforms have been described.

The human ortholog of PAX8 has the amino acid sequence as defined under UniProtKB/Swiss-Prot accession number Q06710. This includes the isoforms defined under accession numbers Q06710-1, Q06710-2, Q06710-3, Q06710-4 and Q06710-5.

Corresponding mRNA sequences include, but are not limited to, the nucleotide sequences defined under accession numbers:
BC001060, XM_011511792.1, NM_013951.3, NM_013952.3, NM_013953.3,
XM_011511790.1, XM_011511793.1, NM_013992.3, NM_003466.3, XM_011511794.1 and XM_011511791.1.

Any nucleotide sequence encoding human PAX8, an ortholog thereof, or a functional fragment thereof, can be used in accordance with this invention. The nucleic acid encoding PAX8 may have a sequence identity of at least 80%, preferably of at least 90%, to the nucleotide sequence as shown in SEQ ID NO:4 [accession number BC001060]. For reprogramming mouse fibroblasts the nucleotide sequence as shown under accession number NM_011040.4 can be used.

The term "overexpression" as used herein means that the expression level of a given gene in a host cell transfected with a nucleic acid encoding the gene is greater than the endogenous expression level of the gene in the non-transfected host cell. For example, if the fibroblasts transfected with an expression vector encoding *Emx2* show a higher expression level of Emx2 nucleic acid than the non-transfected fibroblasts, there is overexpression of *Emx2* in the transfected cells.

The expression level is preferably determined by quantitative Real-Time-PCR.

Fibroblasts may be obtained from the following sources:
Mouse embryonic Fibroblasts may be obtained from the limbs of E11.5-E14.5 embryos.
Postnatal mouse Fibroblasts may be obtained from the tail tips of about 1 week old mice.
Adult mouse fibroblasts may be obtained from the tail tips of about 8 week old mice.
Human Foreskin Fibroblasts may be obtained from the foreskin of a healthy boy, e.g. a 6 year old boy.
Human Fetal Fibroblasts may be obtained from fetal skin (commercially obtainable e.g. from ScienCell Research Laboratories)

In a certain embodiment, the method of the invention comprises only steps carried out *in vitro* and/or *ex vivo.* Accordingly, no step is carried out on the living human or animal body according to this embodiment.

The method of the invention preferably comprises the steps (a) providing one or more nucleic acids encoding EMX2, HNF1b, HNF4a and PAX8; (b) introducing said one or more nucleic acids into the fibroblasts so as to obtain transduced fibroblasts; and (c) culturing said transduced fibroblasts under conditions that allow overexpression of *Emx2, Hnf1b, Hnf4a* and *Pax8.*

The nucleic acids encoding EMX2, HNF1b, HNF4a and PAX8 can be non-circular nucleic acids. Preferably, however, the nucleic acids encoding EMX2, HNF1 b, HNF4a and PAX8 are comprised in a circular nucleic acid, more preferably in a plasmid or a vector. The nucleic acids are typically operably linked to a promoter sequence which is capable of inducing gene expression in fibroblasts. Suitable promoters include, but are not limited to, CMV, EF1a, SV40, PGK1, Ubc, human beta actin, CAG, and TRE.

The nucleic acids encoding EMX2, HNF1b, HNF4a and PAX8 can be on four separate plasmids or vectors. Preferably, however, a plasmid or vector to be used in accordance with this invention comprises nucleotide sequences encoding at least two proteins selected from the group consisting of EMX2, HNF1 b, HNF4a and PAX8. In another embodiment, a plasmid or vector to be used in accordance with this invention comprises nucleotide sequences encoding at least three proteins selected from the group consisting of EMX2, HNF1 b, HNF4a and PAX8. In yet another embodiment, the plasmid or vector to be used in accordance with this invention comprises nucleotide sequences encoding the proteins EMX2, HNF1 b, HNF4a and PAX8. That is, the plasmid or vector comprises coding sequences for all of the four genes *Emx2, Hnf1b, Hnf4a* and *Pax8* or functional equivalent thereof.

The nucleic acid, plasmid or vector can be introduced into the fibroblasts or differentiated cells using techniques that are known *per se.*

Methods suitable for introducing nucleic acids sufficient to achieve expression of a protein of interest into mammalian host cells are known in the art. See, for example, Gething et al., Nature, 293:620-625, 1981; Mantei et al., Nature, 281:40-46, 1979; Levinson et al. EP 117,060; and EP 117,058. For mammalian cells, common methods of introducing genetic material into mammalian cells include the calcium phosphate precipitation method of Graham and van der Erb (Virology, 52:456-457, 1978) or the lipofectamine^{™} (Gibco BRL) Method of Hawley-Nelson (Focus 15:73, 1993). General aspects of mammalian cell host system transformations have been described by Axel in US. Pat. No. 4,399,216. For various techniques for introducing genetic material into mammalian cells, see Keown et al., Methods in Enzymology, 1989, Keown et al., Methods in Enzymology, 185:527-537, 1990, and Mansour et al., Nature, 336:348-352, 1988.

In some embodiments of the methods of the invention, the step of introducing nucleic acid comprises delivering the nucleic acid into the cell (e.g., a human or other animal somatic cell) with a transfection reagent. However, the invention is not limited by the nature of the transfection method utilized. Indeed, any transfection process known, or identified in the future that is able to deliver nucleic acid into cells in vitro or in vivo, is contemplated, including methods that deliver the nucleic acid into cells in culture or in a life-supporting medium, whether said cells comprise isolated cells or cells comprising a eukaryotic tissue or organ, or methods that deliver the nucleic acid in vivo into cells in an organism, such as a human, animal, plant or fungus. In some embodiments, the transfection reagent comprises a lipid (e.g., liposomes, micelles, etc.). In some embodiments, the transfection reagent comprises a nanoparticle or nanotube. In some embodiments, the transfection reagent comprises a cationic compound (e.g., polyethylene imine or PEI). In some embodiments, the transfection method uses an electric current to deliver the mRNA into the cell (e.g., by electroporation).

The nucleic acid can be introduced by transfection or transduction into the cells using a vector, such as an integrating- or non-integrating vector. Of particular interest herein are retroviral vectors. Retroviral vectors, particularly lentiviral vectors, are transduced by packaging the vectors into virions prior to contact with a cell. After introduction, the DNA segment(s) encoding the potency-determining factor(s) can be located extra-chromosomally (e.g., on an episomal plasmid) or stably integrated into cellular chromosome(s).

A viral-based gene transfer and expression vector is a genetic construct that enables efficient and robust delivery of genetic material to most cell types, including non-dividing and hard-to-transfect cells in vitro or in vivo. Viral-based constructs integrated into genomic DNA result in high expression levels. In addition to a DNA segment that encodes a transcription factor of interest, the vectors include a transcription promoter and a polyadenylation signal operatively linked, upstream and downstream, respectively, to the DNA segment. The vector can include a single DNA segment encoding a single potency-determining factor or a plurality of potency-determining factor-encoding DNA segments. A plurality of vectors can be introduced into a single cell. The vector can optionally encode a selectable marker to identify cells that have taken up and express the vector. As an example, when the vector confers antibiotic resistance on the cells, antibiotic can be added to the culture medium to identify successful introduction of the vector into the cells. Integrating vectors can be employed, as in the examples, to demonstrate proof of concept. Retroviral (e.g., lentiviral) vectors are integrating vectors; however, non-integrating vectors can also be used. Such vectors can be lost from cells by dilution after reprogramming, as desired. A suitable non-integrating vector is an Epstein-Barr virus (EBV) vector. Ren C, et al., Acta. Biochim. Biophys. Sin. 37:68-73 (2005); and Ren C, et al., Stem Cells 24:1338-1347 (2006), each of which is incorporated herein by reference as if set forth in its entirety.

The vectors described herein can be constructed and engineered using art-recognized techniques to increase their safety for use in therapy and to include suitable expression elements and therapeutic genes. Standard techniques for the construction of expression vectors suitable for use in the present invention are well-known to one of ordinary skill in the art and can be found in such publications such as Sambrook J, et al., "Molecular cloning: a laboratory manual," (3rd ed. Cold Spring Harbor Press, Cold Spring Harbor, N.Y. 2001), incorporated herein by reference as if set forth in its entirety.

After transfection or transduction, the cells are typically cultured under suitable conditions to allow overexpression of the transfected genes. The culture period from introduction of the gene into the fibroblasts until conversion of the cells into renal cells is typically at least 5 days, preferably at least 10 days, or 5 to 30 days, preferably 10 to 20 days, e.g. 10 days, 11 days, 12 days, 13 days, 14 days, 15 days, 16 days, 17 days, 18 days, 19 days or 20 days.

Suitable culture conditions include, but are not limited to, those described in the examples.

The renal cells obtained by the methods described herein can then be collected or recovered for further use as shown in the examples.

Possible uses include, but are not limited to, nephrotoxicity testing, pharmacological screening or disease modeling. The diseases which can be modeled using the renal cells of the present invention include, but are not limited to, end stage renal disease, polycystic kidney disease, CAKUT, Renal cystic, interstitial and tumorous kidney diseases; Renal tubular and metabolic diseases; and Nephrolithiasis.

### EXAMPLES

### METHODS

### Animals

*Xenopus* embryos were cultured and manipulated as described previously⁴⁶. In brief, female frogs were injected with 700 units of human chorionic gonadotropin (Sigma). On the next morning, oocytes were collected and in vitro fertilized. Embryos were cultured in 0.3x Mark's modified Ringer (1 M NaCl; 20 mM KCI; 10 mM MgCl₂; 20 mM CaCl₂; 50 mM HEPES pH 7.5). Staging was according to Nieuwkoop and Faber (http://xenbase.org). *Qt(ROSA)26Sor*^{*tm4(ACTB-tdTomato,-EGFP)Luo*/*J*} mice were purchased from The Jackson Laboratory (#007676 Strain of origin: B6.129(Cg)) and bred with *KSP-Cre*/*+* (#012237 B6.Cg) mice to label renal tubular epithelial cells. Mice were housed in a SPF facility with free access to chow and water and a 12 h day/night cycle. Breeding and genotyping was done according to standard procedures. All animal experiments were conducted according to the National Institutes of Health *Guide for the Care and Use of Laboratory Animals,* as well as the German law for the welfare of animals and were approved by local authorities (Regierungspräsidium Freiburg X12/08J and X13/08J).

### Cell Culture

Murine embryonic fibroblasts (MEFs) were obtained from limbs of E13 embryos to exclude contamination with renal tissue. Tail tip fibroblasts (TTFs) were obtained from postnatal P7 and adult (P60) mice. Tissue was minced with a scalpel and digested with 0.25% Trypsin-EDTA for 30 min at 37°C. Trypsinization was stopped by addition of MEF Medium (MEFM: Dulbecco's modified Eagle's medium (DMEM), 10% fetal bovine serum (FBS), 2mM L-glutamine, penicillin/streptomycin). After resuspension the cells were centrifuged and the pellet was resuspended in MEFM and plated on gelatine-coated 6-well plates for 3 days until they reached confluency. Postnatal human fibroblasts used for qRT-PCR analysis were derived from foreskin (HFoFs) (Ethics committee number 521/13). Fetal human dermal fibroblasts (HFeF) used for FACS, 2D and 3D immunostainings were obtained from ScienCell Research Laboratories (2300). Human cells were cultivated in DMEM, high glucose, supplemented with 10% fetal bovine serum, 2mM L-glutamine and penicillin/streptomycin. MEFs, TTFs and human fibroblasts were frozen in liquid nitrogen and used without further passaging for reprogramming experiments.

### Albumin uptake assay

Cells were incubated with Alexa647 labeled albumin (1 mg/ml, Life technologies, A34785) at 37°C for 0, 2, 5, 10, 15, 30 and 60 min. After incubation cells were washed five times with ice-cold PBS to stop endocytosis. Cells were fixed with 4% PFA and imaged using a Zeiss Axiovert fluorescence microscope equipped with a 40x lens. The average fluorescence intensity from 5 visual fields per time-point was calculated using ImageJ software.

### Cytotoxicity assay

MEFs and iRECs were harvested after 6, 24 and 36 hours of incubation with 60 µg/ml cisplatin (Teva) by collecting cells in the supernatant as well as attached cells obtained by trypsinization. Cisplatin treated cells and untreated controls were stained with 1 µg/ml DAPI (Invitrogen, D1306) for 5 minutes and fluorescence was measured via flow cytometry using the 405 nm laser for excitation. Dead cells were identified by comparing emission of DAPI stained cell and unstained controls using a 450/50 nm band pass filter. For cisplatin uptake inhibition experiments MEFs and iRECs were left untreated or incubated with either 6 µg/ml cisplatin alone or 6 µg/ml cisplatin and 1mM cimetidine (Sigma-Aldrich, C4522). Cells were harvested and analyzed as described above. Experiments were performed in triplicates and repeated three times.

### 3D cell culture

3D cell culture was performed in Matrigel as described previously⁴⁷. In brief, cells were trypsinized, passed through a 50 µm cell strainer and counted. 10,000 cells were resuspended in 100 µl growth factor reduced Matrigel (Corning, 354230) and seeded in 8-well µ-Slides (Ibidi, 80826). Matrigel was allowed to polymerize for 15 min at 37°C followed by addition of renal epithelial growth medium (Lonza, CC-3190). Cells were imaged after 7 days or when spheroid formation was apparent. For immunofluorescent stainings cells were fixed in 4% PFA.

### Quantitative Real-Time PCR

Total RNA was isolated using RNeasy Universal Plus Kit (Qiagen, 73404). 1 µg of RNA was reverse transcribed using QuantiTect Reverse Transcription Kit (Qiagen, 205311). qRT-PCR was performed using 1/500 to 1/50 of the reverse transcription reaction, gene specific primers and SYBR green Takyon mastermix (Eurogentec, UF-NSCT-B0210) on a ROCHE LC480 light cycler. For each gene three biological replicates were analyzed. Every replicate was measured three times. For data analysis the 2(-Delta C(T)) method was used to calculate relative expression levels followed by statistical analysis using Student's t-test as described previously⁴⁸. The sequences of primers used are shown in Table 1.

**Table 1**

| **List of oligonucleotides used for quantitative RT-PCR analysis** | | |
|---|---|---|
| **Primer name** | **Sequence** | **SEQ ID NO:** |
| *Esrrg* Forward | CTATGGGGTTGCATCATGTGAA | 5 |
| *Esrrg* Reverse | CGTCTGCGCTTTGTGATCTC | 6 |
| *Hnf1a* Forward | GACCTGACCGAGTTGCCTAAT | 7 |
| *Hnf1a* Reverse | CCGGCTCTTTCAGAATGGGT | 8 |
| *Lhx1* Forward | CCCATCCTGGACCGTTTCC | 9 |
| *Lhx1* Reverse | CGCTTGGAGAGATGCCCTG | 10 |
| *Nr1h4* Forward | GCTTGATGTGCTACAAAAGCTG | 11 |
| *Nr1h4* Reverse | CGTGGTGATGGTTGAATGTCC | 12 |
| *Pax2* Forward | AAGCCCGGAGTGATTGGTG | 13 |
| *Pax2* Reverse | CAGGCGAACATAGTCGGGTT | 14 |
| *Tfcp2l1* Forward | GCTGGAGAATCGGAAGCTAGG | 15 |
| *Tfcp2l1* Reverse | AAAACGACACGGATGATGCTC | 16 |
| *Vdr* Forward | GTGCAGCGTAAGCGAGAGAT | 17 |
| *Vdr* Reverse | GGATGGCGATAATGTGCTGTTG | 18 |
| *Abcc2* Forward | GTGTGGATTCCCTTGGGCTTT | 19 |
| *Abcc2* Reverse | CACAACGAACACCTGCTTGG | 20 |
| *Ggt1* Forward | TTTGTCATCATCGGCCTCTGT | 21 |
| *Ggt1* Reverse | CCCGTCCAATCTCTGAGCAG | 22 |
| *Lrp2* Forward | ACACTTGTGGGCATTCTC | 23 |
| *Lrp2* Reverse | GCAGTCATTATCACCATCAC | 24 |
| *Slc6a18* Forward | TGCTTTGCCTGTTTCCTCTCA | 25 |
| *Slc6a18* Reverse | ATGTCATCACAGAACCGTTTCAT | 26 |
| *Slc17a1* Forward | CCAAGGACCACCCGTATATGA | 27 |
| *Slc17a1* Reverse | CTTTGATTGGCAGGGATTGTCT | 28 |
| *Slc23a1* Forward | AAAGCAGCATGAGGTCGTGG | 29 |
| *Slc23a1* Reverse | ACTGAAGCACGTCAGGTAATG | 30 |
| *Trpv4* Forward | GCCGATTGAAGACTTTGAGG | 31 |
| *Trpv4* Reverse | CTACGGCACTTACCGTCACC | 32 |
| *Cdh1* Forward | AAGTACATCCTCTATTCTCA | 33 |
| *Cdh1* Reverse | ATTCTGATCTGTCACTGT | 34 |
| *Cdh6* Forward | GCAACGGAGATTAATAACC | 35 |
| *Cdh6* Reverse | TCATAGAACTCAGCAAACT | 36 |
| *Cdh16* Forward | ATACCAACTACAGGTCAC | 37 |
| *Cdh16* Reverse | GTCATTCTCATCTTTCACAT | 38 |
| *Actb* Forward | CCTTCTTGGGTATGGAATC | 39 |
| *Actb* Reverse | CACTGTGTTGGCATAGAG | 40 |
| *Tbp* Forward | AGAACAATCCAGACTAGCAGCA | 41 |
| *Tbp* Reverse | GGGAACTTCACATCACAGCTC | 42 |
| *HNF1A* Forward | AACACCTCAACAAGGGCACTC | 43 |
| *HNF1A* Reverse | CCCCACTTGAAACGGTTCCT | 44 |
| *NR1H4* Forward | GACTTTGGACCATGAAGACCAG | 45 |
| *NR1H4* Reverse | GCCCAGACGGAAGTTTCTTATT | 46 |
| *TFCP2L1* Forward | CGTTTAAGCAGAACGAGAATGGG | 47 |
| *TFCP2L1* Reverse | TTTCATAGGACGGCTGGTATTTC | 48 |
| *CDH16* Forward | AGAATGACAACGTGCCTATCTG | 49 |
| *CDH16* Reverse | GCTGACAGTCTAGTCACTTCAGT | 50 |
| *EPCAM* Forward | ATAACCTGCTCTGAGCGAGTG | 51 |
| *EPCAM* Reverse | TGCAGTCCGCAAACTTTTACTA | 52 |
| *GGT1* Forward | CTGGGGAGATCCGAGGCTAT | 53 |
| *GGT1* Reverse | GATGACGGTCCGCTTGTTTTC | 54 |
| *LRP2* Forward | GGCCTGCTATAACACCAGTCA | 55 |
| *LRP2* Reverse | ACTCATTGTGCAAGCATATCTCA | 56 |
| *SLC17A1* Forward | TCTGTTCCTTTCGCTATGGATTG | 57 |
| *SLC17A1* Reverse | TTGGGCAAACCATGTGGATCT | 58 |
| *HSPCB* Forward | TCTGGGTATCGGAAAGCAAGCC | 59 |
| *HSPCB* Reverse | GTGCACTTCCTCAGGCATCTTG | 60 |

### Whole-mount in situ hybridization

Whole-mount *in situ* hybridization of *Xenopus* embryos was performed at stages 22, 26, 33 and 38. Digoxigenin-UTP labeled antisense probes were used as described before⁴⁶. To generate *in situ* probes, plasmids were linearized and transcribed with T7 or SP6 (Roche, DIG labeling kit). Antibody against digoxigenin conjugated to alkaline phosphatase was used to detect bound probes (Roche, 11093274910). *In situ* hybridization of paraffin-embedded mouse kidney sections was performed as described previously⁴⁹. In brief, embryos, or dissected kidney anlagen were fixed in 4% paraformaldehyde (PFA)/phosphate buffered saline (PBS), dehydrated through ethanol series and embedded in paraffin for sectioning at 10 µm. Digoxigenin-UTP labeled antisense probes were used for *in situ* hybridization and Eosin counterstaining was performed according to standard protocols⁴⁹. Plasmids used for probe synthesis are available upon request from the authors.

### Colony Formation Assay

Fifty cells from MEFs or iRECs were seeded in 6-well plates and allowed to grow for 14 days prior to fixation and staining with 0.1% crystal violet in a 10% ethanol solution for 15 min at room temperature. Colonies were counted by macroscopic visual inspection of cells from 3 independent experiments.

### Microarray Analyses

Total RNA from CFP treated control MEFs, GFP sorted iRECs and GFP sorted primary renal KSP-Cre tubule cells was isolated using RNeasy Universal Plus Kit (Qiagen, 73404). All cells were subjected to sorting prior to RNA extraction. Total RNA samples were processed with the Affymetrix WT Plus kit (Ambion, Austin TX, USA) as described by the manufacturer. Labeled fragments were hybridized to Affymetrix WT Mouse Gene ST 1.0/2.0 arrays for 16 h at 45°C and 60 rpm in an Affymetrix hybridization oven 645. After washing and staining using the Hybridization, Wash and Stain Kit (Affymetrix, USA), the arrays were scanned with the Affymetrix GeneChip Scanner 3000 7G resulting in signal intensity values for each individual probe. CEL files were generated from the raw data with Affymetrix GeneChip Command Console Software Version 4.0. We used Partek Genomics Suite software for further analysis (Partek, Inc.). CEL files were imported including control and interrogating probes. Pre-background adjustment was set to adjust for GC Content and probe sequence and RMA background correction was performed. Arrays were normalized using Quantile normalization and probeset summarization was done using Median Polish. Probe values were log2 transformed. In order to identify differentially expressed genes between the groups we performed a 1-way ANOVA in Partek. We used Fisher's Least Significant Difference (LSD) as Contrast method. The array results are deposited at ArrayExpress (E-MTAB-3648).

### Molecular cloning and lentiviral transduction

Full-length or fragments of cDNA *Xenopus* and mouse transcription factors were cloned from cDNA of reverse transcribed total RNA isolates from stage 39 *Xenopus* and E15 mouse embryonic kidneys. Gene specific primers were used to amplify the coding sequences of the transcription factors. PCR products were cloned into pWPXLd, (Addgene (#12258)). To produce lentiviruses, plasmid DNA, pMD2.G (Addgene #12559), and psPax2 (Addgene #12260) were transfected into 293T cells using Calcium Phosphate transfection. Two days after transfection cell supernatants were harvested and virus was concentrated using polyethylene glycol precipitation as described previously⁵⁰. Concentrated virus was frozen at -80°C and thawed directly before transduction of cells. For the infection of MEFs, TTFs, HFoFs and HFeFs virus concentrate was diluted 1:100 to 1:1000 in MEFM containing 10 µg/ml Polybrene and cells were incubated for 12 h. Viral infection was repeated five times. After cultivation in MEFM for 7 days, MEFs and TTFs were analyzed for GFP expression by flow cytometry. GFP positive iRECs were isolated by fluorescence activated cell sorting (FACS) 14 days after the last virus transduction and expanded for further analysis. Reprogrammed HFeFs were identified 28 days after the last transduction by sorting for CD90⁻ CD326⁺ cells.

### Western blot analysis

Proteins were fractionated by SDS/PAGE and anti 2A-peptide antibody (ABS31, Merck Millipore) was used for protein detection by Western blot.

### Immunofluorescent stainings

Cells were washed twice with PBS and fixed in 4% paraformaldehyde for 15 min at room temperature. For permeabilization the cells were incubated with PBST (0.1% Triton-X-100 in PBS) for 10 min at room temperature. After blocking for 1 h with 2% horse serum, 5% BSA, 1% goldfish gelatin in PBS cells were incubated with primary antibody for 60 min at room temperature and subsequently with a secondary fluorescence-conjugated antibody for 60 min at room temperature in the dark. DNA was stained with Hoechst 33342 diluted 1:2,000 in PBS. Primary and secondary antibodies were diluted in PBS containing 10% of the blocking solution. Antibodies used for immunofluorescent staining were as follows: β-Catenin (Santa-Cruz, sc7199, 1:100), E-Cadherin (Invitrogen, 13-1900, 1:100), Epcam (Abcam, ab71916, 1:100), Laminin (Sigma, L9393, 1:100), LTL (Biozol, B-1325, 1:100), Megalin (Santa-Cruz, sc16478, 1:100), Na⁺K⁺-ATPase (Abcam, ab7671, 1:100), PAX2 (Abcam, ab37129, 1:100), Phalloidin (Mol. Probes, A22287, 1:100), TRPV4 (Alomone, acc-034, 1:100), Vimentin (Sigma, V2258), ZO-1 (Santa-Cruz, sc33725, 1:100).

The following secondary antibodies were used: Goat anti rat Alexa-633 (Life Technologies, A-21094, 1:500), Goat anti rabbit Alexa-647 (Life Technologies, A-21245, 1:500), Goat anti mouse Alexa-647 (Life Technologies, A-21235, 1:500), Donkey anti goat Alexa-647 (Life Technologies, A-21447, 1:500)

### Flow cytometry

For flow cytometry analyses, cells were harvested, resuspended in FACS buffer (PBS supplemented with 3% FBS and 5 mM EDTA), filtered through a 50 µm cell strainer and analyzed by the 13-color LCR Fortessa FACS analyzer (Becton Dickinson). For the selection of GFP positive cells the ARIA III cell sorter (Becton Dickinson) was used. Data were analyzed by FlowJo (FlowJo LLC) or FACS DIVA (Becton Dickinson) software. For sorting of CD90⁻ CD326⁺ mouse and human cells the following antibodies were used according to the manufacturer's instructions: anti-mouse CD90 Alexa 647 (Biolegend, 105317), anti-mouse CD326 BV421 (BD Bioscience, 563214), anti-human CD90 FITC (MACS Miltenyi Biotec, 130-097-930), anti-human CD326 BV421 (BD Bioscience, 563180).

### Kidney reaggregation

Kidney reaggregation was performed as described previously⁵¹. In brief, 20 E13 embryonic mouse kidneys were harvested and digested. 80,000 embryonic kidney cells were mixed with 8,000 iRECs or control cells and pelleted by centrifugation. The pellet was transferred to a low-binding PCR tube and allowed to aggregate overnight at 37°C. Next day the cell aggregates were transferred to a transwell filter (Costar, 3450) and cultured for 4 days at 37°C, 5% CO₂. Reaggregates were fixed in 4% PFA, stained and imaged by confocal microscopy.

### Recellularization of decellularized kidneys

For recellularization experiments⁴⁰ cadaveric kidneys from adult wild-type rats were flushed 10 times with heparinized PBS (10 U/ml; Sigma H3393) followed by incubation in 1% SDS (Roth 2326.3) in PBS for three days until decellularization was observed. During this time kidneys were repeatedly flushed with 1% SDS in PBS every 6 to 12 h. Subsequently, decellularized kidneys were rinsed 5 times with PBS containing penicillin/ streptomycin and 100 µg/ml Normocin (Invivogen). Seeding of iRECs into the organ scaffolds was performed by injection of 10⁶ trypsinized cells with a 25G needle and subsequent cultivation in MEFM for 14 days.

### Imaging

Confocal imaging was performed using a LSM-I-NLO2 510 META microscope equipped with a 25x/0.8 LD LCI-Plan-Apochromat objective (both Carl Zeiss). Excitation of the fluorophores (Hoechst 33342, GFP, Tomato, Alexa647) was performed with a two photon laser at 740 nm, and a single photon laser at 488 nm, 561 nm and 633 nm, respectively. Image analysis was performed using ZEN (Zeiss) and Imaris (Bitplane) software.

### Transmission electron microscopy

3D cultures of MEFs and iRECs were fixed in 4% PFA and 1% glutaraldehyde in 0.1 M phosphate buffer for 1 h at room temperature. After washing in 0.1 M PB cultures were contrasted using 1% OsO₄ (Sigma-Aldrich, Germany) for 45 minutes and 1% uranyl acetate (Polysciences, Germany) in 70% ethanol. After dehydration cultures were flat embedded in Durcupan (Sigma-Aldrich, Germany). Ultrathin sections (40 nm thickness) were analyzed using a Philipps CM 100 transmission electron microscope.

### Data analysis

Quantitative expression data²² for all transcription factors across various human tissues was analyzed to identify candidate reprogramming factors. Relative expression was determined as the absolute expression in the target tissue divided by the median of all other tissues. Transcriptional circuitry analysis was performed as previously described³³ and the online platform http://www.regulatorynetworks.org/ was used to retrieve predicted transcriptional targets of reprogramming factors. Differential expression values between MEFs and iRECs of the microarray experiments were mapped to each target and visualized using Cytoscape⁵². CellNet scores were calculated by the online platform http://cellnet.hms.harvard.edu/ ³⁰. For comparison of differentially regulated genes to published RNAseq data, the raw data^{31, 32} was mapped to 10 fold differentially regulated genes between iREC and MEFs based on microarray analysis. For GO-term analysis of molecular function, 10 fold differentially regulated genes were analyzed using the DAVID-platform⁵³. Statistical analysis was performed using SigmaStat and graphs were made with GraphPadPrism. Data analysis was performed with Microsoft Excel.

### RESULTS

### Identification of Renal Cell Fate Inducing Transcription Factors

To define criteria for the unbiased selection of candidate kidney reprogramming factors (Fig. 1a), we analyzed quantitative mRNA expression data of all known transcription factors across different adult human tissues²² (Fig. 1b). Previously reported reprogramming factors were expressed at high absolute levels in their target tissue and showed high relative expression in comparison to other tissues. Based on these criteria a set of 55 candidate kidney reprogramming factors could be defined.

Core regulators of renal identity are likely to have an evolutionary conserved function during early nephrogenesis. Central aspects of *Xenopus laevis* pronephros development and function exhibit remarkable similarities to mammalian kidney development²³. Therefore, we performed a comparative *in situ* hybridization screen of orthologous genes to the 55 human candidate factors in *Xenopus* (Fig. 1c and 8). 38 genes showed a distinct expression in the pronephros; 18 were detected at the earliest analyzed time point (stage 22). Comparable early and tissue-restricted expression during mouse metanephros development was detected in embryonic day 12 (E12) to E17 kidney anlagen (Fig. 1c) and by comparison to published expression data ²⁴⁻²⁶. Thus, 38 factors with early and evolutionary conserved expression during renal development were included in subsequent analysis.

As a third selection criterion for potential reprogramming factors, genes associated with congenital renal disease in humans or renal loss-of-function phenotypes in mouse were considered essential and non-redundant for renal development. This stepwise selection further reduced the number of potential reprogramming factors to 13 candidates that were functionally tested for their potency to induce renal cell fate.

### Emx2, Hnf1b, Hnf4a and Pax8 Convert Fibroblasts into iRECs.

First, we tested if individual or combinations of candidate reprogramming factors could direct mammalian cells towards renal cell fate. Because candidate factors were predicted based on expression data from whole kidneys, we focused on tubule epithelial cells that constitute the major cell type of the kidney.

To detect a fate switch towards tubule cell identity, we crossed mice harboring Cre-recombinase under control of the renal tubule-specific cadherin-16 promoter (Cdh16/Ksp-Cre (kidney specific protein)) to mice with a membrane-tandem dimer tomato red/membrane-GFP (mTOM/mGFP) dual fluorescent reporter²⁷. In resulting mice Cre-mediated recombination leads to the expression of membrane GFP specifically in renal tubular epithelial cells, while non Cre-expressing cells maintain red fluorescence (Fig. 2a). We isolated mouse embryonic fibroblasts (MEFs) from limbs of E13 embryos carrying the reporter system to exclude potential contamination with renal precursor cells. Next, we expressed candidate transcription factors in isolated MEFs by lentiviral transduction and analyzed GFP reporter-expression by flow cytometry (Fig. 2a and 9a).

When a cocktail of all 13 candidate factors was transduced, 0.1% of cells expressed GFP one week after transduction (Fig. 2b). To determine the minimal set of factors required for kidney-fate induction, we systematically removed individual candidates from the pool of 13 transcription factors (13TF-1; Fig. 2c). Withdrawal of *Foxc1*, *Gata3, Hnf1a, Lhx1,* and *Tfap2b* increased the percentage of GFP-positive cells. Consequently, these factors were excluded from the following experiments (8TF-1; Fig. 2d). Removal of each of the four factors *Emx2, Hnf1b, Hnf4a,* and *Pax8*, either from the pools of 13 or 8 factors significantly reduced the percentage of GFP-positive cells (Figs. 2c,d). Conversely, the combined expression of these four factors significantly increased the average percentage of GFP-positive cells to 0.6%.

Importantly, none of the four factors could induce reporter activity when overexpressed individually (Figs. 2e,f). Of note, the percentage of GFP-positive cells increased to 11% five weeks after lentiviral transduction (Figs. 2g,h).

Next, we tested, if the expression of all four transcription factors from a single vector would be sufficient for reprogramming. Multicistronic expression ensures defined stoichiometric expression and can enhance reprogramming efficiency for *in vivo* applications²⁸. Here, on average 5.4% of cells were GFP-positive 39 days after lentiviral transduction (Figs. 9b,c,d), suggesting that the multicistronic expression of four factors can induce cell type conversion. To evaluate if postnatal fibroblasts could similarly be used for reprogramming, tail-tip fibroblasts of postnatal day 7 (P7) and adult mice were transduced with four transcription factors. The detection of GFP-positive cells indicated that reprogramming towards renal epithelial fate was not limited to embryonic fibroblasts (Figs. 9e,f).

In conclusion, a combination of four factors (i.e. *Emx2, Hnf1b, Hnf4a, Pax8*) is sufficient to activate renal tubule specific reporter expression in embryonic, and postnatal fibroblasts. We therefore termed these cells induced renal tubular epithelial cells (iRECs).

### iRECs Show Epithelial Characteristics

To characterize iRECs, GFP-positive cells were isolated by fluorescence-activated cell sorting (FACS) and expanded in culture. iRECs exhibited a characteristic epithelial morphology and formed a tight epithelial layer when grown to confluency (Fig. 3a). Sparsely seeded cells formed colonies after 2 weeks of culture, suggesting that iRECs have the capacity of clonogenic proliferation¹⁷ (Figs. 3b,c). iRECs cultured in serum-reduced renal epithelial-growth medium on Matrigel-coated dishes formed branched epithelial structures (Fig. 3d). Densely grown iRECs frequently exhibited dome-like structures (Fig. 3d), a characteristic feature of renal epithelial cells indicative of directed transport of solutes and water influx²⁹.

Next, we analyzed epithelial cell adhesion molecules with a strong expression in renal tubule cells. Both *Cdh1* and *Cdh6* transcripts, and as expected from Ksp-Cre reporter activation, *Cdh16* (Ksp) were robustly expressed in iRECs (Fig. 9g).

In addition, immunostaining for ZO-1, E-cadherin (CDH1) and EPCAM demonstrated membrane localization of these epithelial marker proteins in iRECs, but not in MEFs (Fig. 3e). In contrast, the intermediate filament protein vimentin, marking mesenchymal cells, was present in MEFs, but absent in iRECs. Thus, iRECs acquired epithelial cell identity during reprogramming, and had generally lost their mesenchymal phenotype.

### iRECs Resemble Primary Tubule Cells in their Expression Profile.

Next, we assessed the global transcriptional profile of iRECs in comparison to FACS-isolated primary renal tubule cells from P7 Ksp-Cre mTOM/mGFP mice and MEFs. Hierarchical clustering analysis of microarray data indicated that iRECs more closely resembled primary renal tubular epithelial cells than MEFs (Fig. 4a).

Recently, a quantitative measure for the degree to which engineered cells resemble their target tissue was introduced (CellNet)³⁰. Calculation of this classification score is based on gene regulatory network activation and extends from 0 (no similarity) to 1 (indistinguishable). CellNet analysis revealed that iRECs more closely resemble kidney tissue than any other cell types (Fig. 4b), except for some residual fibroblast signature. iRECs possessed a mean classification score of 0.30, similar to the score of directly converted neurons (0.34) and cardiomyocytes (0.27) (Fig. 4c). Thus, the degree of similarity of iRECs to their native counterparts matches other previous reprogramming approaches.

We further assessed the expression of kidney-specific marker genes by qRT-PCR (Fig. 4d). The transcriptional regulators of kidney development *Pax2* or *Lhx1,* the highly kidney specific amino acid transporter Slc6a18, the organic anion transporter *Slcl7a1* and the receptor protein *Lrp2* were strongly upregulated, confirming the microarray expression analysis.

To investigate the main transcriptional changes during reprogramming to iRECs, expression levels of genes that were differentially regulated in our dataset were analyzed in various mouse and human tissues using published RNA-sequencing expression datasets^{31, 32}. Genes that were 10-fold upregulated in iRECs versus MEFs were most abundantly expressed in kidney samples, while down-regulated genes were strongly expressed in MEFs (Fig. 10a). Gene ontology (GO) analysis revealed that categories associated with transmembrane transport activity were significantly enriched in 10-fold upregulated genes (Fig. 10b), consistent with the elevated expression of tubular expressed ion channels and transporters in iRECs (Fig. 4d). We concluded that the mRNA expression profile of iRECs is most similar to that of renal tissue.

To explore how the combinatorial actions of the 4TFs regulate target gene expression and initiate the transcriptional circuitry of iRECs, we retrieved 164 putative direct target genes of the four reprogramming factors from published gene-regulatory networks³³ and analyzed their expression levels in iRECs in comparison to MEFs (Fig. 4e). Interestingly, prominent regulators of renal tubule development, such as *Pax2, Lhx1,* and *Hnf1a* were strongly induced targets of at least one of the four reprogramming factors. The nephron progenitor marker *Osr1* was down-regulated in iRECs and primary tubule cells compared to MEFs, suggesting that iRECs represent differentiated cells rather than renal progenitors. Collectively, these analyses indicate that the combinatorial action of the four reprogramming factors regulate the expression of a limited set of downstream transcription factors that may additionally contribute to the reprogramming and maintenance of iRECs.

Next, we sought to determine if high mRNA transcript levels of renal tubular genes in iRECs were also reflected by protein expression. The alphal subunit of the Na⁺/K⁺-ATPase (ATP1A1) contributes to blood pressure regulation by controlling sodium excretion³⁴. ATP1A1 was detected at the lateral membrane in iRECs, consistent with its subcellular localization and function *in vivo* (Fig. 4f). Immunostaining for the transcription factor PAX2 showed nuclear staining, whereas Aquaporin 1 (AQP1) was detected at the membrane (Fig. 4f). Thus, upregulated mRNA in iRECs resulted in translation and correct subcellular targeting of the analyzed proteins.

### iRECs Display Morphological and Functional Characteristics of Renal Tubular Epithelial Cells.

Next, we characterized the behavior of iRECs in a three dimensional extracellular matrix. iRECs consistently formed spheres with a central lumen when grown in Matrigel 3D culture (Figs. 5a,b,c). In contrast, MEFs only formed irregular and unstructured conglomerates without obvious organization. iREC-derived spheres exhibited a robust apico-basal polarization with apical localization of tight junction protein ZO-1, apical actin accumulation indicated by phalloidin staining, and basolateral localization of β-catenin (Fig. 5d). Similarly, the localization of Na⁺/K⁺-ATPase alpha1 was detected basolaterally in iRECs, while megalin (LRP2) showed apical localization (Fig. 5d), consistent with their subcellular localization in renal tubular epithelial cells. The mechanosensory channel TRPV4 was detected predominantly at the apical membrane. Staining for the mesenchymal marker vimentin was completely absent from iREC spheres (Fig. 5d).

In addition, we performed ultrastructural analysis of 3D spheres by transmission electron microscopy. iRECs appeared as polarized cuboidal epithelial cells, established complex tight junctions and extended multiple luminal microvilli from their apical surface (Fig. 5e). Spheres were surrounded by a tight and electron-dense extracellular matrix, reminiscent of basement membrane material (Fig. 5e). The apolipoprotein-receptor megalin (LRP2) mediates the endocytotic uptake of proteins, including albumin³⁵. Based on its prominent expression, we tested iRECs for their uptake of fluorescently labeled albumin. Fluorescence intensity was significantly increased in iRECs compared to MEFs (Figs. 5f,g), indicating that iRECs possess endocytotic activity.

Cisplatin, a commonly used chemotherapeutic drug, has major nephrotoxic side effects by damaging renal tubular cells³⁶. To test whether iRECs exhibit cisplatin sensitivity we incubated MEFs and iRECs with 60 µM cisplatin for up to 36 h. Apoptosis was significantly increased in Cisplatin treated iRECs in comparison to MEFs (Fig. 5h). Cisplatin uptake into renal tubular cells is mediated in part by the organic cation transporter 2 (Oct2)³⁷. Notably, cisplatin induced toxicity in iRECs could be decreased by incubation with 1 mM of Cimetidine, a known inhibitor of Oct2³⁸ (Fig. 5i). Collectively, the functional features of iRECs are reminiscent of renal tubular epithelial cells and indicate that iRECs can be employed for renal toxicity testing.

### iRECs Integrate into Renal Organoids and Form Tubular Structures in Decellularized Kidneys.

Single cell suspensions of embryonic kidney cells can reaggregate and self-organize into renal organoids including embryonic renal structures such as ureteric bud and early tubules³⁹. To test if iRECs share this potential, E13 embryonic kidneys constitutively expressing mTomato were disintegrated to single-cell suspension, and mixed with GFP-expressing iRECs or MEFs, followed by reaggregation and culture (Fig. 6a). While MEFs failed to integrate into tubular structures and were predominantly found in the stroma or were even excluded from the aggregates, iRECs integrated into tubule epithelial structures (Fig. 6b). Immunostaining for laminin demonstrated that iRECs shared a continuous basement membrane layer with neighboring epithelial cells (Fig. 6c). The proximal tubule marker LTL and megalin were similarly found in iRECs and in native tubule cells demonstrating that iRECs were differentiated and polarized within the renal microenvironment (Fig. 6c). Thus, iRECs can participate in the formation of self-organizing kidney organoids.

Finally, we asked if iRECs would also assemble into tubules in the absence of other cell types, only guided by the structure of extracellular matrix (ECM). Decellularized kidneys with intact three-dimensional ECM were thus used as guidance scaffold⁴⁰. iRECs grew into long, and partially convoluted tubules within decellularized kidneys (Fig. 6d). Thus, iRECs have the potential to repopulate a kidney scaffold along preformed guidance structures in the absence of other cell types.

### Induction of human renal epithelial cells.

Next we tested if human fibroblasts could also be reprogrammed towards a renal cell fate. Combined overexpression of *EMX2, HNF1B, HNF4A,* and *PAX8* in human fibroblasts resulted in cells with epithelial morphology similar to murine iRECs (Fig. 7a). 4TF treated human cells showed marker expression comparable to iRECs, including membranous β-Catenin, nuclear PAX2, and positive staining for proximal tubule specific LTL (Fig. 7a). mRNA levels of renal transcription factors, adhesion molecules and transporters were upregulated after 4TF treatment as compared to fibroblasts (Fig. 7b).

Due to the lack of a genetically encoded reporter to indicate renal tubule cell fate in human cells, we enriched reprogrammed human fibroblast based on their expression of epithelial markers. Expression of 4TFs in both mouse and human cells reduced the fibroblasts marker THY-1 (CD90) as determined by flow cytometry (Fig. 7c). In contrast, the epithelial cell adhesion molecule EPCAM (CD326) was upregulated (Fig. 7c) both in mouse iRECs and human reprogrammed cells. We therefore enriched human iRECs (h-iRECs) by sorting for CD90⁻ CD326⁺ four weeks after lentiviral infection (Fig. 7d). Sorted h-iRECs formed spheroids in Matrigel, similar to their mouse counterparts (Fig. 7e). The spheroids were polarized, as indicated by apical actin accumulation and basolateral β-Catenin expression (Fig. 7f). While the frequency of lumen formation was lower and the overall size of human derived spheroids was smaller in comparison to mouse iRECs, the expression of *EMX2, HNF1B*, *HNF4A,* and *PAX8* could similarly reprogram human fibroblasts into renal epithelial like cells (h-iRECs).

### REFERENCES:

1. USRD United States Renal Data System. Annual Data Report: Epidemiology of Kidney Disease in the United States. National Institutes of Health, National Institute of Diabetes and Digestive and Kidney Diseases, Bethesda, MD. (2014).
2. Morizane, R. et al. Nephron organoids derived from human pluripotent stem cells model kidney development and injury. Nat Biotech advance online publication (2015).
3. Taguchi, A. et al. Redefining the in vivo origin of metanephric nephron progenitors enables generation of complex kidney structures from pluripotent stem cells. Cell Stem Cell 14, 53-67 (2014).
4. Takasato, M. et al. Directing human embryonic stem cell differentiation towards a renal lineage generates a self-organizing kidney. Nat Cell Biol 16, 118-126 (2014).
5. Takasato, M. et al. Kidney organoids from human iPS cells contain multiple lineages and model human nephrogenesis. Nature advance online publication (2015).
6. Xia, Y. et al. Directed differentiation of human pluripotent cells to ureteric bud kidney progenitor-like cells. Nat Cell Biol 15, 1507-1515 (2013).
7. Papadimou, E. et al. Direct Reprogramming of Human Bone Marrow Stromal Cells into Functional Renal Cells Using Cell-free Extracts. Stem Cell Reports (2015).
8. Knoepfler, P.S. Deconstructing stem cell tumorigenicity: a roadmap to safe regenerative medicine. Stem Cells 27, 1050-1056 (2009).
9. Xu, J., Du, Y. & Deng, H. Direct lineage reprogramming: strategies, mechanisms, and applications. Cell Stem Cell 16, 119-134 (2015).
10. Vierbuchen, T. et al. Direct conversion of fibroblasts to functional neurons by defined factors. Nature 463, 1035-1041 (2010).
11. Najm, F.J. et al. Transcription factor-mediated reprogramming of fibroblasts to expandable, myelinogenic oligodendrocyte progenitor cells. Nat Biotechnol 31, 426-433 (2013).
12. Yang, N. et al. Generation of oligodendroglial cells by direct lineage conversion. Nat Biotechnol 31, 434-439 (2013).
13. leda, M. et al. Direct reprogramming of fibroblasts into functional cardiomyocytes by defined factors. Cell 142, 375-386 (2010).
14. Du, Y. et al. Human hepatocytes with drug metabolic function induced from fibroblasts by lineage reprogramming. Cell Stem Cell 14, 394-403 (2014).
15. Huang, P. et al. Induction of functional hepatocyte-like cells from mouse fibroblasts by defined factors. Nature 475, 386-389 (2011).
16. Sekiya, S. & Suzuki, A. Direct conversion of mouse fibroblasts to hepatocyte-like cells by defined factors. Nature 475, 390-393 (2011).
17. Buganim, Y. et al. Direct reprogramming of fibroblasts into embryonic Sertoli-like cells by defined factors. Cell Stem Cell 11, 373-386 (2012).
18. Hendry, C.E. et al. Direct transcriptional reprogramming of adult cells to embryonic nephron progenitors. J Am Soc Nephrol 24, 1424-1434 (2013).
19. Heinaniemi, M. et al. Gene-pair expression signatures reveal lineage control. Nat Methods 10, 577-583 (2013).
20. Pereira, C.F., Lemischka, I.R. & Moore, K. Reprogramming cell fates: insights from combinatorial approaches. Ann N Y Acad Sci 1266, 7-17 (2012).
21. Lang, A.H., Li, H., Collins, J.J. & Mehta, P. Epigenetic landscapes explain partially reprogrammed cells and identify key reprogramming genes. PLoS Comput Biol 10, e1003734 (2014).
22. Ravasi, T. et al. An atlas of combinatorial transcriptional regulation in mouse and man. Cell 140, 744-752 (2010).
23. Raciti, D. et al. Organization of the pronephric kidney revealed by large-scale gene expression mapping. Genome Biol 9, R84 (2008).
24. Harding, S.D. et al. The GUDMAP database--an online resource for genitourinary research. Development 138, 2845-2853 (2011).
25. McMahon, A.P. et al. GUDMAP: the genitourinary developmental molecular anatomy project. J Am Soc Nephrol 19, 667-671 (2008).
26. Yu, J. et al. Identification of molecular compartments and genetic circuitry in the developing mammalian kidney. Development 139, 1863-1873 (2012).
27. Shao, X., Somlo, S. & Igarashi, P. Epithelial-specific Cre/lox recombination in the developing kidney and genitourinary tract. JAm Soc Nephrol 13, 1837-1846 (2002).
28. Riddell, J. et al. Reprogramming committed murine blood cells to induced hematopoietic stem cells with defined factors. Cell 157, 549-564 (2014).
29. Valentich, J.D., Tchao, R. & Leighton, J. Hemicyst formation stimulated by cyclic AMP in dog kidney cell line MDCK. J Cell Physiol 100, 291-304 (1979).
30. Cahan, P. et al. CellNet: network biology applied to stem cell engineering. Cell 158, 903-915 (2014).
31. Cabili, M.N. et al. Integrative annotation of human large intergenic noncoding RNAs reveals global properties and specific subclasses. Genes Dev 25, 1915-1927 (2011).
32. Shen, Y. et al. A map of the cis-regulatory sequences in the mouse genome. Nature 488, 116-120 (2012).
33. Neph, S. et al. Circuitry and dynamics of human transcription factor regulatory networks. Cell 150, 1274-1286 (2012).
34. Jaitovich, A. & Bertorello, A.M. Salt, Na+,K+-ATPase and hypertension. Life Sci 86, 73-78 (2010).
35. Cui, S., Verroust, P.J., Moestrup, S.K. & Christensen, E.I. Megalin/gp330 mediates uptake of albumin in renal proximal tubule. Am J Physiol 271, F900-907 (1996).
36. Pabla, N. & Dong, Z. Cisplatin nephrotoxicity: mechanisms and renoprotective strategies. Kidney international 73, 994-1007 (2008).
37. Ciarimboli, G. et al. Cisplatin nephrotoxicity is critically mediated via the human organic cation transporter 2. The American journal of pathology 167, 1477-1484 (2005).
38. Yonezawa, A. et al. Association between tubular toxicity of cisplatin and expression of organic cation transporter rOCT2 (Slc22a2) in the rat. Biochemical pharmacology 70, 1823-1831 (2005).
39. Unbekandt, M. & Davies, J.A. Dissociation of embryonic kidneys followed by reaggregation allows the formation of renal tissues. Kidney Int 77, 407-416 (2010).
40. Song, J.J. et al. Regeneration and experimental orthotopic transplantation of a bioengineered kidney. Nat Med 19, 646-651 (2013).
41. Nam, Y.J. et al. Induction of diverse cardiac cell types by reprogramming fibroblasts with cardiac transcription factors. Development 141, 4267-4278 (2014).
42. Wernig, M. et al. A drug-inducible transgenic system for direct reprogramming of multiple somatic cell types. Nat Biotechnol 26, 916-924 (2008).
43. Yoo, A.S. et al. MicroRNA-mediated conversion of human fibroblasts to neurons. Nature 476, 228-231 (2011).
44. Murphy, S.V. & Atala, A. 3D bioprinting of tissues and organs. Nat Biotechnol 32, 773-785 (2014).
45. Lau, S., Rylander Ottosson, D., Jakobsson, J. & Parmar, M. Direct neural conversion from human fibroblasts using self-regulating and nonintegrating viral vectors. Cell Rep 9, 1673-1680 (2014).
46. Lienkamp, S. et al. Inversin relays Frizzled-8 signals to promote proximal pronephros development. Proc Natl Acad Sci U S A 107, 20388-20393 (2010).
47. Giles, R.H., Ajzenberg, H. & Jackson, P.K. 3D spheroid model of mIMCD3 cells for studying ciliopathies and renal epithelial disorders. Nat Protoc 9, 2725-2731 (2014).
48. Schmittgen, T.D. & Livak, K.J. Analyzing real-time PCR data by the comparative C(T) method. Nat Protoc 3, 1101-1108 (2008).
49. Nagy, A.G., M.; Vintersten, K.; Behringer, R. [Production of chimeras]. Manipulating the mouse embryo. A laboratory manual. 3rd edition., in Cold Spring Harbor Laboratory Press 453-506 (2003).
50. Kutner, R.H., Zhang, X.Y. & Reiser, J. Production, concentration and titration of pseudotyped HIV-1-based lentiviral vectors. Nat Protoc 4, 495-505 (2009).
51. Xia, Y. et al. The generation of kidney organoids by differentiation of human pluripotent cells to ureteric bud progenitor-like cells. Nat Protoc 9, 2693-2704 (2014).
52. Shannon, P. et al. Cytoscape: a software environment for integrated models of biomolecular interaction networks. Genome Res 13, 2498-2504 (2003).
53. Huang da, W., Sherman, B.T. & Lempicki, R.A. Systematic and integrative analysis of large gene lists using DAVID bioinformatics resources. Nat Protoc 4, 44-57 (2009).

## Claims

1. A method for producing renal cells, comprising overexpressing *Emx2, Hnf1b, Hnf4a* and *Pax8* in differentiated cells.

2. The method of claim 1, wherein the renal cells are renal tubular cells.

3. The method of claim 1, wherein the renal cells are renal tubular epithelial cells.

4. The method of any one of the preceding claims, wherein the differentiated cells are fibroblasts.

5. The method of claim 4, wherein the fibroblasts are embryonic fibroblasts.

6. The method of claim 4, wherein the fibroblasts are adult fibroblasts.

7. The method of any one of the preceding claims, wherein said overexpressing comprises (a) providing one or more nucleic acids encoding Emx2, Hnf1b, Hnf4a and Pax8; (b) introducing said one or more nucleic acids into the differentiated cells so as to obtain transduced or transfected cells; and (c) culturing said transduced or transfected cells under conditions that allow overexpression of *Emx2, Hnf1b, Hnf4a* and *Pax8.*

8. The method of claim 7, wherein said one or more nucleic acids are plasmids or vectors comprising a nucleotide sequence encoding *Emx2, Hnf1b, Hnf4a, Pax8* or a combination thereof, operably linked to a promoter capable of inducing overexpression of the encoded genes.

9. The method of any one of the preceding claims, wherein said *Emx2* comprises the nucleotide sequence as shown in SEQ ID NO:1 or a functional fragment thereof.

10. The method of any one of the preceding claims, wherein said *Hnf1b* comprises the nucleotide sequence as shown in SEQ ID NO:2 or a functional fragment thereof.

11. The method of any one of the preceding claims, wherein said *Hnf4a* comprises the nucleotide sequence as shown in SEQ ID NO:3 or a functional fragment thereof.

12. The method of any one of the preceding claims, wherein said *Pax8* comprises the nucleotide sequence as shown in SEQ ID NO:4 or a functional fragment thereof.

13. A renal cell obtainable by the method of any one of the preceding claims.

14. The use of the renal cell of claim 13 for nephrotoxicity testing, pharmacological screening or disease modeling.


SEQUENCE LISTING

<110>  Albert-Ludwigs-Universitaet Freiburg

<120>  Method of Producing Renal Cells from Fibroblasts

<130>  PEP02669ALB

<160>  60

<170>  PatentIn version 3.5

<210>  1
<211>  2908
<212>  DNA
<213>  Homo sapiens

<400>  1
cgggcgccgc aggagcgagt gagctgggag cgaggggcga aggcgcggag aagcccggcc      60

gcccggtggg cggcagaagg ctcagccgag gcggcggcgc cgactccgtt ccactctcgg     120

cccggatcca ggcctccggg ttcccaggcg ctcacctccc tctgacgcac tttaaagagt     180

ctcccccctt ccacctcagg gcgagtaata gcgaccaatc atcaagccat ttaccaggct     240

tcggaggaag ctgtttatgt gatccccgca ctaattaggc tcatgaacta acaaatcgtt     300

tgcacaactt gtgaagaagc gaacacttcc atggattgtc cttggactta gggcgccctg     360

cccgcctttt gcagaggaga aaaaactttt tttttttttt gcctcccccg agaactttcc     420

ccccttctcc tccctgcctc taactccgat ccccccacgc catctcgcca aaaaaaaaaa     480

aaaaaaaaaa aaagaaaaaa aaagaaaaaa aaagaaaaaa aattacccca atccacgcct     540

gcaaattctt ctggaaggat tttcccccct ctcttcaggt tgggcgcgtt tggtgcaaga     600

ttctcgggat cctcggcttt gcctctccct ctccctcccc cctcctttcc tttttccttt     660

cctttccttt ctttcttcct ttccttcccc ccacccccac ccccacccca aacaaacgag     720

tccccaattc tcgtccgtcc tcgccgcggg cagcgggcgg cggaggcagc gtgcggcggt     780

cgccaggagc tgggagccca gggcgcccgc tcctcggcgc agcatgttcc agccggcgcc     840

caagcgctgc ttcaccatcg agtcgctggt ggccaaggac agtcccctgc ccgcctcgcg     900

ctccgaggac cccatccgtc ccgcggcact cagctacgct aactccagcc ccataaatcc     960

gttcctcaac ggcttccact cggccgccgc cgccgccgcc ggtaggggcg tctactccaa    1020

cccggacttg gtgttcgccg aggcggtctc gcacccgccc aaccccgccg tgccagtgca    1080

cccggtgccg ccgccgcacg ccctggccgc ccacccccta ccctcctcgc actcgccaca    1140

ccccctattc gcctcgcagc agcgggatcc gtccaccttc tacccctggc tcatccaccg    1200

ctaccgatat ctgggtcatc gcttccaagg gaacgacact agccccgaga gtttcctttt    1260

gcacaacgcg ctggcccgaa agcccaagcg gatccgaacc gccttctccc cgtcccagct    1320

tctaaggctg gaacacgcct ttgagaagaa tcactacgtg gtgggcgccg aaaggaagca    1380

gctggcacac agcctcagcc tcacggaaac tcaggtaaaa gtatggtttc agaaccgaag 1440

aacaaagttc aaaaggcaga agctggagga agaaggctca gattcgcaac aaaagaaaaa 1500

agggacgcac catattaacc ggtggagaat cgccaccaag caggcgagtc cggaggaaat 1560

agacgtgacc tcagatgatt aaaaacataa acctaacccc acagaaacgg acaacatgga 1620

gcaaaagaga cagggagagg tggagaagga aaaaacccta caaaacaaaa acaaaccgca 1680

tacacgttca ccgagaaagg gagagggaat cggagggagc agcggaatgc ggcgaagact 1740

ctggacagcg agggcacagg gtcccaaacc gaggccgcgc caagatggca gaggatggag 1800

gctccttcat caacaagcga ccctcgtcta aagaggcagc tgagtgagag acacagagag 1860

aaggagaaag agggagggag agagagaaag agagagaaag agagagagag agagagagag 1920

agaaagctga acgtgcactc tgacaagggg agctgtcaat caaacaccaa accggggaga 1980

caagatgatt ggcaggtatt ccgtttatca cagtccactt aaaaaatgat gatgatgata 2040

aaaaccacga cccaaccagg cacaggactt ttttgttttt tgcacttcgc tgtgtttccc 2100

ccccatcttt aaaaataatt agtaataaaa aacaaaaatt ccatatctag ccccatccca 2160

cacctgtttc aaatccttga aatgcatgta gcagttgttg ggcgaatggt gtttaaagac 2220

cgaaaatgaa ttgtaatttt cttttccttt taaagacagg ttctgtgtgc tttttatttt 2280

gatttttttt cccaagaaat gtgcagtctg taaacacttt ttgatacctt ctgatgtcaa 2340

agtgattgtg caagctaaat gaagtaggct cagcgatagt ggtcctctta cagagaaacg 2400

gggagcagga cgacgggggg gctgggggtg gcgggggagg gtgcccacaa aaagaatcag 2460

gacttgtact gggaaaaaaa cccctaaatt aattatattt cttggacatt ccctttccta 2520

acatcctgag gcttaaaacc ctgatgcaaa cttctccttt cagtggttgg agaaattggc 2580

cgagttcaac cattcactgc aatgcctatt ccaaacttta aatctatcta ttgcaaaacc 2640

tgaaggactg tagttagcgg ggatgatgtt aagtgtggcc aagcgcacgg cggcaagttt 2700

tcaagcactg agtttctatt ccaagatcat agacttacta aagagagtga caaatgcttc 2760

cttaatgtct tctataccag aatgtaaata tttttgtgtt ttgtgttaat ttgttagaat 2820

tctaacacac tatatacttc caagaagtat gtcaatgtca atattttgtc aataaagatt 2880

tatcaatatg ccctcaaaaa aaaaaaaa 2908

<210> 2
<211> 2818
<212> DNA
<213> Homo sapiens

<400> 2
atcatggcaa gttagaagtt ttctgactcc tttcggagga gcctccggga ccccggggag 60

taacaggtgt ctggaggctg aagggtggag gggttcctgg atttggggtt tgcttgtgaa 120

actcccctcc accctcctct ctcgcaccca cccaccccct cacccccttc tttttccgtc 180

cttggaaaat ggtgtccaag ctcacgtcgc tccagcaaga actcctgagc gccctgctga 240

gctccggggt caccaaggag gtgctggttc aggccttgga ggagttgctg ccatccccga 300

acttcggggt gaagctggag acgctgcccc tgtcccctgg cagcggggcc gagcccgaca 360

ccaagccggt cttccatact ctcaccaacg gccacgccaa gggccgcttg tccggcgacg 420

agggctccga ggacggcgac gactatgaca cacctcccat cctcaaggag ctgcaggcgc 480

tcaacaccga ggaggcggcg gagcagcggg cggaggtgga ccggatgctc agtgaggacc 540

cttggagggc tgctaaaatg atcaagggtt acatgcagca acacaacatc ccccagaggg 600

aggtggtcga tgtcaccggc ctgaaccagt cgcacctctc ccagcatctc aacaagggca 660

cccctatgaa gacccagaag cgtgccgctc tgtacacctg gtacgtcaga aagcaacgag 720

agatcctccg acaattcaac cagacagtcc agagttctgg aaatatgaca gacaaaagca 780

gtcaggatca gctgctgttt ctctttccag agttcagtca acagagccat gggcctgggc 840

agtccgatga tgcctgctct gagcccacca acaagaagat gcgccgcaac cggttcaaat 900

gggggcccgc gtcccagcaa atcttgtacc aggcctacga tcggcaaaag aaccccagca 960

aggaagagag agaggcctta gtggaggaat gcaacagggc agaatgtttg cagcgagggg 1020

tgtccccctc caaagcccac ggcctgggct ccaacttggt cactgaggtc cgtgtctaca 1080

actggtttgc aaaccgcagg aaggaggagg cattccggca aaagctggcc atggacgcct 1140

atagctccaa ccagactcac agcctgaacc ctctgctctc ccacggctcc ccccaccacc 1200

agcccagctc ctctcctcca aacaagctgt caggagtgcg ctacagccag cagggaaaca 1260

atgagatcac ttcctcctca acaatcagtc accatggcaa cagcgccatg gtgaccagcc 1320

agtcggtttt acagcaagtc tccccagcca gcctggaccc aggccacaat ctcctctcac 1380

ctgatggtaa aatgatctca gtctcaggag gaggtttgcc cccagtcagc accttgacga 1440

atatccacag cctctcccac cataatcccc agcaatctca aaacctcatc atgacacccc 1500

tctctggagt catggcaatt gcacaaagcc tcaacacctc ccaagcacag agtgtccctg 1560

tcatcaacag tgtggccggc agcctggcag ccctgcagcc cgtccagttc tcccagcagc 1620

tgcacagccc tcaccagcag cccctcatgc agcagagccc aggcagccac atggcccagc 1680

agcccttcat ggcagctgtg actcagctgc agaactcaca catgtacgca cacaagcagg 1740

aaccccccca gtattcccac acctcccggt ttccatctgc aatggtggtc acagatacca 1800

gcagcatcag tacactcacc aacatgtctt caagtaaaca gtgtcctcta caagcctggt 1860

gatgcccaca caccacttac ttcgtgcgca acaacaagga ccctgttttc cacaccatca 1920

ccctctgggc agctgtcatg gaaaagccca gtgacctgac cagcacctgc gagaggtccc 1980

tgcttacctg acggacgtcc tgctggcacc tcagacaatc cactctcagg aggcgcagcc 2040

cgaagcccag tttcccttct atgcagtatt gccacaatgc ctctcccacg atgtcaagga 2100

ctcctgtctg tcctggaggt gggagacaag gaacctccga agaggaagca agaaagccgt 2160

actgtctatg ttgtgatcct tcatcgaaca aactgatgcg aaaacttgaa tctgttactg 2220

aaatgaggag agaaggacat gtgctattga actgagccaa acacactgta aatatccaca 2280

gactccctcc cctgccccca tcccaaatga tcttgagatt tcttttaaag aagtaaattt 2340

gtccaatggc tgtaaactat aaactactgt aattaagtgc aatttcccct ctgtgtcctc 2400

tcccctctgc cctgtatata atactaaagt gtctattagt tttctttgta aaggtcagag 2460

tcaaaatttc aaaagtgatc tgtcccctct cccctcatgg agaaacatcc taagtgggaa 2520

gtgaagcccc ttgtcctctc ccgcgggcct ggacacttat ggggacagca taccttggac 2580

tgactaccag ctaactccag tctcctgaca ttaagacaca cctctggatc cctggagggg 2640

ctgaatgtag tgtgtcagag taacatgcca gcttcctgtg ggccaggagc tcagccgtgc 2700

actccctaag aaaccccagg gcagggaaac tggctgtttg atagcagaag aaaaagttgc 2760

agtctcagaa agccttccat taaaacaatt tattttatca ctaaaaaaaa aaaaaaaa 2818

<210> 3
<211> 4737
<212> DNA
<213> Homo sapiens

<400> 3
ggtttgaaag gaaggcagag agggcactgg gaggaggcag tgggagggcg gagggcgggg 60

gccttcgggg tgggcgccca gggtagggca ggtggccgcg gcgtggaggc agggagaatg 120

cgactctcca aaaccctcgt cgacatggac atggccgact acagtgctgc actggaccca 180

gcctacacca ccctggaatt tgagaatgtg caggtgttga cgatgggcaa tgacacgtcc 240

ccatcagaag gcaccaacct caacgcgccc aacagcctgg gtgtcagcgc cctgtgtgcc 300

atctgcgggg accgggccac gggcaaacac tacggtgcct cgagctgtga cggctgcaag 360

ggcttcttcc ggaggagcgt gcggaagaac cacatgtact cctgcagatt tagccggcag 420

tgcgtggtgg acaaagacaa gaggaaccag tgccgctact gcaggctcaa gaaatgcttc 480

cgggctggca tgaagaagga agccgtccag aatgagcggg accggatcag cactcgaagg 540

tcaagctatg aggacagcag cctgccctcc atcaatgcgc tcctgcaggc ggaggtcctg 600

tcccgacaga tcacctcccc cgtctccggg atcaacggcg acattcgggc gaagaagatt 660

gccagcatcg cagatgtgtg tgagtccatg aaggagcagc tgctggttct cgttgagtgg 720

gccaagtaca tcccagcttt ctgcgagctc cccctggacg accaggtggc cctgctcaga 780

gcccatgctg gcgagcacct gctgctcgga gccaccaaga gatccatggt gttcaaggac 840

gtgctgctcc taggcaatga ctacattgtc cctcggcact gcccggagct ggcggagatg 900
agccgggtgt ccatacgcat ccttgacgag ctggtgctgc ccttccagga gctgcagatc 960
gatgacaatg agtatgccta cctcaaagcc atcatcttct ttgacccaga tgccaagggg 1020
ctgagcgatc cagggaagat caagcggctg cgttcccagg tgcaggtgag cttggaggac 1080
tacatcaacg accgccagta tgactcgcgt ggccgctttg gagagctgct gctgctgctg 1140
cccaccttgc agagcatcac ctggcagatg atcgagcaga tccagttcat caagctcttc 1200
ggcatggcca agattgacaa cctgttgcag gagatgctgc tgggagggtc ccccagcgat 1260
gcaccccatg cccaccaccc cctgcaccct cacctgatgc aggaacatat gggaaccaac 1320
gtcatcgttg ccaacacaat gcccactcac ctcagcaacg gacagatgtg tgagtggccc 1380
cgacccaggg gacaggcagc cacccctgag accccacagc cctcaccgcc aggtggctca 1440
gggtctgagc cctataagct cctgccggga gccgtcgcca caatcgtcaa gcccctctct 1500
gccatccccc agccgaccat caccaagcag gaagttatct agcaagccgc tggggcttgg 1560
gggctccact ggctcccccc agccccctaa gagagcacct ggtgatcacg tggtcacggc 1620
aaaggaagac gtgatgccag gaccagtccc agagcaggaa tgggaaggat gaagggcccg 1680
agaacatggc ctaaggccca catcccactg ccacccttga cgccctgctc tggataacaa 1740
gactttgact tggggagacc tctactgcct tggacaactt ttctcatgtt gaagccactg 1800
ccttcacctt caccttcatc catgtccaac ccccgacttc atcccaaagg acagccgcct 1860
ggagatgact tgaggcctta cttaaaccca gctcccttct tccctagcct ggtgcttctc 1920
ctctcctagc ccctgtcatg gtgtccagac agagccctgt gaggctgggt ccaattgtgg 1980
cacttggggc accttgctcc tccttctgct gctgccccca cctctgctgc ctccctctgc 2040
tgtcaccttg ctcagccatc ccgtcttctc caacaccacc tctccagagg ccaaggaggc 2100
cttggaaacg attcccccag tcattctggg aacatgttgt aagcactgac tgggaccagg 2160
caccaggcag ggtctagaag gctgtggtga gggaagacgc ctttctcctc caacccaacc 2220
tcatcctcct tcttcaggga cttgggtggg tacttgggtg aggatccctg aaggccttca 2280
acccgagaaa acaaacccag gttggcgact gcaacaggaa cttggagtgg agaggaaaag 2340
catcagaaag aggcagacca tccaccaggc ctttgagaaa gggtagaatt ctggctggta 2400
gagcaggtga gatgggacat tccaaagaac agcctgagcc aaggcctagt ggtagtaaga 2460
atctagcaag aattgaggaa gaatggtgtg ggagagggat gatgaagaga gagagggcct 2520
gctggagagc atagggtctg gaacaccagg ctgaggtcct gatcagcttc aaggagtatg 2580
cagggagctg ggcttccaga aaatgaacac agcagttctg cagaggacgg gaggctggaa 2640
gctgggaggt caggtggggt ggatgatata atgcgggtga gagtaatgag gcttggggct 2700
ggagaggaca agatgggtaa accctcacat cagagtgaca tccaggagga ataagctccc 2760

agggcctgtc tcaagctctt ccttactccc aggcactgtc ttaaggcatc tgacatgcat 2820

catctcattt aatcctccct tcctccctat taacctagag attgtttttg ttttttattc 2880

tcctcctccc tccccgccct cacccgcccc actccctcct aacctagaga ttgttacaga 2940

agctgaaatt gcgttctaag aggtgaagtg attttttttc tgaaactcac acaactagga 3000

agtggctgag tcaggacttg aacccaggtc tccctggatc agaacaggag ctcttaacta 3060

cagtggctga atagcttctc caaaggctcc ctgtgttctc accgtgatca agttgagggg 3120

cttccggctc ccttctacag cctcagaaac cagactcgtt cttctgggaa ccctgcccac 3180

tcccaggacc aagattggcc tgaggctgca ctaaaattca cttagggtcg agcatcctgt 3240

ttgctgataa atattaagga gaattcatga ctcttgacag cttttctctc ttcactcccc 3300

aagtcaaggg gaggggtggc aggggtctgt ttcctggaag tcaggctcat ctggcctgtt 3360

ggcatggggg tgggacagtg tgcacagtgt gggggcaggg gagggctaag caggcctggg 3420

tttgagggct gctccggaga ccgtcactcc aggtgcattc tggaagcatt agaccccagg 3480

atggagcgac cagcatgtca tccatgtgga atcttggtgg ctttgaggac attctggaaa 3540

atgccactga ccagtgtgaa caaaagggat gtgttatggg gctggaggtg tgattaggta 3600

ggagggaaac tgttggaccg actcctgccc cctgctcaac actgacccct ctgagtggtt 3660

ggaggcagtg ccccagtgcc cagaaatccc accattagtg attgtttttt atgagaaaga 3720

ggcgtggaga agtattgggg caatgtgtca gggaggaatc accacatccc tacggcagtc 3780

ccagccaagc ccccaatccc agcggagact gtgccctgct cagagctccc aagccttccc 3840

ccaccacctc actcaagtgc ccctgaaatc cctgccagac ggctcagcct ggtctgcggt 3900

aaggcaggga ggctggaacc atttctgggc attgtggtca ttcccactgt gttcctccac 3960

ctcctccctc cagcgttgct cagacctctg tcttgggaga aaggttgaga taagaatgtc 4020

ccatggagtg ccgtgggcaa cagtggccct tcatgggaac aatctgttgg agcagggggt 4080

cagttctctg ctgggaatct acccctttct ggaggagaaa cccattccac cttaataact 4140

ttattgtaat gtgagaaaca caaaacaaag tttacttttt tgactctaag ctgacatgat 4200

attagaaaat ctctcgctct cttttttttt tttttttttt tttttggcta cttgagttgt 4260

ggtcctaaaa cataaaatct gatggacaaa cagagggttg ctggggggac aagcgtgggc 4320

acaatttccc caccaagaca ccctgatctt caggcgggtc tcaggagctt ctaaaaatcc 4380

gcatggctct cctgagagtg gacagaggag aggagagggt cagaaatgaa cgctcttcta 4440

tttcttgtca ttaccaagcc aattactttt gccaaatttt tctgtgatct gccctgatta 4500

agatgaattg tgaaatttac atcaagcaat tatcaaagcg ggctgggtcc catcagaacg 4560

acccacatct ttctgtgggt gtgaatgtca ttaggtcttg cgctgacccc tgagccccca 4620

tcactgccgc ctgatggggc aaagaaacaa aaaacatttc ttactcttct gtgttttaac 4680

aaaagtttat aaaacaaaat aaatggcgca tatgttttct aaaaaaaaaa aaaaaaa 4737

<210> 4
<211> 2698
<212> DNA
<213> Homo sapiens

<400> 4
   cttcagaagg aggagagaca ccgggcccag ggcaccctcg cgggcggacc caagcagtga 60
   gggcctgcag ccggccggcc agggcagcgg caggcgcggc ccggacctac gggaggaagc 120
   cccgagccct cggcgggctg cgagcgactc cccggcgatg cctcacaact ccatcagatc 180
   tggccatgga gggctgaacc agctgggagg ggcctttgtg aatggcagac ctctgccgga 240
   agtggtccgc cagcgcatcg tagacctggc ccaccagggt gtaaggccct gcgacatctc 300
   tcgccagctc cgcgtcagcc atggctgcgt cagcaagatc cttggcaggt actacgagac 360
   tggcagcatc cggcctggag tgataggggg ctccaagccc aaggtggcca cccccaaggt 420
   ggtggagaag attggggact acaaacgcca gaaccctacc atgtttgcct gggagatccg 480
   agaccggctc ctggctgagg gcgtctgtga caatgacact gtgcccagtg tcagctccat 540
   taatagaatc atccggacca aagtgcagca accattcaac ctccctatgg acagctgcgt 600
   ggccaccaag tccctgagtc ccggacacac gctgatcccc agctcagctg taactccccc 660
   ggagtcaccc cagtcggatt ccctgggctc cacctactcc atcaatgggc tcctgggcat 720
   cgctcagcct ggcagcgaca agaggaaaat ggatgacagt gatcaggata gctgccgact 780
   aagcattgac tcacagagca gcagcagcgg accccgaaag caccttcgca cggatgcctt 840
   cagccagcac cacctcgagc cgctcgagtg cccatttgag cggcagcact acccagaggc 900
   ctatgcctcc cccagccaca ccaaaggcga gcagggcctc tacccgctgc ccttgctcaa 960
   cagcaccctg gacgacggga aggccaccct gaccccttcc aacacgccac tggggcgcaa 1020
   cctctcgact caccagacct accccgtggt ggcagatcct cactcaccct tcgccataaa 1080
   gcaggaaacc cccgaggtgt ccagttctag ctccacccct tcctctttat ctagctccgc 1140
   ctttttggat ctgcagcaag tcggctccgg ggtcccgccc ttcaatgcct ttccccatgc 1200
   tgcctccgtg tacgggcagt tcacgggcca ggccctcctc tcagggcgag agatggtggg 1260
   gcccacgctg cccggatacc caccccacat ccccaccagc ggacagggca gctatgcctc 1320
   ctctgccatc gcaggcatgg tggcaggaag tgaatactct ggcaatgcct atggccacac 1380
   cccctactcc tcctacagcg aggcctggcg cttccccaac tccagcttgc tgagttcccc 1440
   atattattac agttccacat caaggccgag tgcaccgccc accactgcca cggcctttga 1500
   ccatctgtag ttgccatggg gacagtggga gcgactgagc aacaggagga ctcagcctgg 1560
   gacaggcccc agagagtcac acaaaggaat ctttatttat tacatgaaaa ataaccacaa 1620
   gtccagcatt gcggcacact ccctgtgtgg ttaatttaat gaaccatgaa agacaggatg 1680
   accttggaca aggccaaact gtcctccaag actccttaat gaggggcagg agtcccaggg 1740
   aaagagaacc atgccatgct gaaaaagaca aaattgaaga agaaatgtag cccccagccg 1800
   gtacccacca aaggagagaa gaagcaatag ccgaggaact tggggggatg gcgaatggtt 1860
   cctgcccggg cccaaggggt gcacagggca cctccatggc tccattatta acacaactct 1920
   agcaattatg gaccataagc acttccctcc agcccacaag tcacagcctg gtgccgaggc 1980
   tctcctcacc agccacccag ggagtcacct ccctcagcct cccgcctgcc ccacacggag 2040
   gctctggctg tcctctttct ccactccatt tgcttggctc tttctacacc tccctcttgg 2100
   gcatgggctg agggctggag cgagtccctc agaaattcca ccaggctgtc agctgacctc 2160
   ttttgcctgc tgctgtgaag gtatagcacc accccaggtc ctcctgcagt gcggcatccc 2220
   cttggcagct gccgtcagcc aggccagccc cagggagctt aaaacagaca ttccacaggg 2280
   cctgggcccc tgggaggtga ggtgtggtgt gcggcttcac ccagggcaga acaaggcaga 2340
   atcgcaggaa acccgcttcc ccttcctgac agctcctgcc aagccaaatg tgcttcctgc 2400
   agctcacgcc caccagctac tgaagggacc caaggcaccc cctgaagcca gcgatagagg 2460
   gtccctctct gctccccagc agctcctgcc cccaaggcct gactgtatat actgtcaatg 2520
   aaactttgtt tgggtcaagc ttccttcttt ctacccccca gactttggcc tctgagtgaa 2580
   atgtctctct ttgccctgtg gggcttctct ccttgatgct tctttctttt tttaaagaca 2640
   acctgccatt accacatgac tcaataaacc attgctcttc aaaaaaaaaa aaaaaaaa 2698

<210> 5
<211> 22
<212> DNA
<213> Artificial Sequence

<220>
<223> Primer

<400> 5
ctatggggtt gcatcatgtg aa 22

<210> 6
<211> 20
<212> DNA
<213> Artificial Sequence

<220>
<223> Primer

<400> 6
cgtctgcgct ttgtgatctc 20

<210>  7
<211>  21
<212>  DNA
<213>  Artificial Sequence

<220>
<223>  Primer

<400>  7
gacctgaccg agttgcctaa t                                                21


<210>  8
<211>  20
<212>  DNA
<213>  Artificial Sequence

<220>
<223>  Primer

<400>  8
ccggctcttt cagaatgggt                                                  20


<210>  9
<211>  19
<212>  DNA
<213>  Artificial Sequence

<220>
<223>  Primer

<400>  9
cccatcctgg accgtttcc                                                   19


<210>  10
<211>  19
<212>  DNA
<213>  Artificial Sequence

<220>
<223>  Primer

<400>  10
cgcttggaga gatgccctg                                                   19


<210>  11
<211>  22
<212>  DNA
<213>  Artificial Sequence

<220>
<223>  Primer

<400>  11
gcttgatgtg ctacaaaagc tg                                               22


<210>  12
<211>  21
<212>  DNA

<213>  Artificial Sequence

<220>
<223>  Primer

<400>  12
cgtggtgatg gttgaatgtc c                                                21


<210>  13
<211>  19
<212>  DNA
<213>  Artificial Sequence

<220>
<223>  Primer

<400>  13
aagcccggag tgattggtg                                                   19


<210>  14
<211>  20
<212>  DNA
<213>  Artificial Sequence

<220>
<223>  Primer

<400>  14
caggcgaaca tagtcgggtt                                                  20


<210>  15
<211>  21
<212>  DNA
<213>  Artificial Sequence

<220>
<223>  Primer

<400>  15
gctggagaat cggaagctag g                                                21


<210>  16
<211>  21
<212>  DNA
<213>  Artificial Sequence

<220>
<223>  Primer

<400>  16
aaaacgacac ggatgatgct c                                                21


<210>  17
<211>  20
<212>  DNA
<213>  Artificial Sequence

<220>

<223>  Primer

<400>  17
gtgcagcgta agcgagagat                                                    20


<210>  18
<211>  22
<212>  DNA
<213>  Artificial Sequence

<220>
<223>  Primer

<400>  18
ggatggcgat aatgtgctgt tg                                                 22


<210>  19
<211>  21
<212>  DNA
<213>  Artificial Sequence

<220>
<223>  Primer

<400>  19
gtgtggattc ccttgggctt t                                                  21


<210>  20
<211>  20
<212>  DNA
<213>  Artificial Sequence

<220>
<223>  Primer

<400>  20
cacaacgaac acctgcttgg                                                    20


<210>  21
<211>  21
<212>  DNA
<213>  Artificial Sequence

<220>
<223>  Primer

<400>  21
tttgtcatca tcggcctctg t                                                  21


<210>  22
<211>  20
<212>  DNA
<213>  Artificial Sequence

<220>
<223>  Primer

<400>  22

cccgtccaat ctctgagcag                                                20

<210>  23
<211>  18
<212>  DNA
<213>  Artificial Sequence

<220>
<223>  Primer

<400>  23
acacttgtgg gcattctc                                                  18

<210>  24
<211>  20
<212>  DNA
<213>  Artificial Sequence

<220>
<223>  Primer

<400>  24
gcagtcatta tcaccatcac                                                20

<210>  25
<211>  21
<212>  DNA
<213>  Artificial Sequence

<220>
<223>  Primer

<400>  25
tgctttgcct gtttcctctc a                                              21

<210>  26
<211>  23
<212>  DNA
<213>  Artificial Sequence

<220>
<223>  Primer

<400>  26
atgtcatcac agaaccgttt cat                                            23

<210>  27
<211>  21
<212>  DNA
<213>  Artificial Sequence

<220>
<223>  Primer

<400>  27
ccaaggacca cccgtatatg a                                              21

<210>  28
<211>  22
<212>  DNA
<213>  Artificial Sequence

<220>
<223>  Primer

<400>  28
ctttgattgg cagggattgt ct                                                22


<210>  29
<211>  20
<212>  DNA
<213>  Artificial Sequence

<220>
<223>  Primer

<400>  29
aaagcagcat gaggtcgtgg                                                   20


<210>  30
<211>  21
<212>  DNA
<213>  Artificial Sequence

<220>
<223>  Primer

<400>  30
actgaagcac gtcaggtaat g                                                 21


<210>  31
<211>  20
<212>  DNA
<213>  Artificial Sequence

<220>
<223>  Primer

<400>  31
gccgattgaa gactttgagg                                                   20


<210>  32
<211>  20
<212>  DNA
<213>  Artificial Sequence

<220>
<223>  Primer

<400>  32
ctacggcact taccgtcacc                                                   20


<210>  33
<211>  20
<212>  DNA

<213>  Artificial Sequence

<220>
<223>  Primer

<400>  33
aagtacatcc tctattctca                                                   20


<210>  34
<211>  18
<212>  DNA
<213>  Artificial Sequence

<220>
<223>  Primer

<400>  34
attctgatct gtcactgt                                                     18


<210>  35
<211>  19
<212>  DNA
<213>  Artificial Sequence

<220>
<223>  Primer

<400>  35
gcaacggaga ttaataacc                                                    19


<210>  36
<211>  19
<212>  DNA
<213>  Artificial Sequence

<220>
<223>  Primer

<400>  36
tcatagaact cagcaaact                                                    19


<210>  37
<211>  18
<212>  DNA
<213>  Artificial Sequence

<220>
<223>  Primer

<400>  37
ataccaacta caggtcac                                                     18


<210>  38
<211>  20
<212>  DNA
<213>  Artificial Sequence

<220>

<223>  Primer

<400>  38
gtcattctca tctttcacat                                                    20


<210>  39
<211>  19
<212>  DNA
<213>  Artificial Sequence

<220>
<223>  Primer

<400>  39
ccttcttggg tatggaatc                                                     19


<210>  40
<211>  18
<212>  DNA
<213>  Artificial Sequence

<220>
<223>  Primer

<400>  40
cactgtgttg gcatagag                                                      18


<210>  41
<211>  22
<212>  DNA
<213>  Artificial Sequence

<220>
<223>  Primer

<400>  41
agaacaatcc agactagcag ca                                                 22


<210>  42
<211>  21
<212>  DNA
<213>  Artificial Sequence

<220>
<223>  Primer

<400>  42
gggaacttca catcacagct c                                                  21


<210>  43
<211>  21
<212>  DNA
<213>  Artificial Sequence

<220>
<223>  Primer

<400>  43

aacacctcaa caagggcact c 21

<210> 44
<211> 20
<212> DNA
<213> Artificial Sequence

<220>
<223> Primer

<400> 44
ccccacttga aacggttcct 20

<210> 45
<211> 22
<212> DNA
<213> Artificial Sequence

<220>
<223> Primer

<400> 45
gactttggac catgaagacc ag 22

<210> 46
<211> 22
<212> DNA
<213> Artificial Sequence

<220>
<223> Primer

<400> 46
gcccagacgg aagtttctta tt 22

<210> 47
<211> 23
<212> DNA
<213> Artificial Sequence

<220>
<223> Primer

<400> 47
cgtttaagca gaacgagaat ggg 23

<210> 48
<211> 23
<212> DNA
<213> Artificial Sequence

<220>
<223> Primer

<400> 48
tttcatagga cggctggtat ttc 23

<210>  49
<211>  22
<212>  DNA
<213>  Artificial Sequence

<220>
<223>  Primer

<400>  49
agaatgacaa cgtgcctatc tg                                                22


<210>  50
<211>  23
<212>  DNA
<213>  Artificial Sequence

<220>
<223>  Primer

<400>  50
gctgacagtc tagtcacttc agt                                               23


<210>  51
<211>  21
<212>  DNA
<213>  Artificial Sequence

<220>
<223>  Primer

<400>  51
ataacctgct ctgagcgagt g                                                 21


<210>  52
<211>  22
<212>  DNA
<213>  Artificial Sequence

<220>
<223>  Primer

<400>  52
tgcagtccgc aaacttttac ta                                                22


<210>  53
<211>  20
<212>  DNA
<213>  Artificial Sequence

<220>
<223>  Primer

<400>  53
ctggggagat ccgaggctat                                                   20


<210>  54
<211>  21
<212>  DNA

<213>  Artificial Sequence

<220>
<223>  Primer

<400>  54
gatgacggtc cgcttgtttt c                                                 21


<210>  55
<211>  21
<212>  DNA
<213>  Artificial Sequence

<220>
<223>  Primer

<400>  55
ggcctgctat aacaccagtc a                                                 21


<210>  56
<211>  23
<212>  DNA
<213>  Artificial Sequence

<220>
<223>  Primer

<400>  56
actcattgtg caagcatatc tca                                               23


<210>  57
<211>  23
<212>  DNA
<213>  Artificial Sequence

<220>
<223>  Primer

<400>  57
tctgttcctt tcgctatgga ttg                                               23


<210>  58
<211>  21
<212>  DNA
<213>  Artificial Sequence

<220>
<223>  Primer

<400>  58
ttgggcaaac catgtggatc t                                                 21


<210>  59
<211>  22
<212>  DNA
<213>  Artificial Sequence

<220>

<223>  Primer

<400>  59
tctgggtatc ggaaagcaag cc                                                22


<210>  60
<211>  22
<212>  DNA
<213>  Artificial Sequence

<220>
<223>  Primer

<400>  60
gtgcacttcc tcaggcatct tg                                                22